# EUROPEAN PATENT APPLICATION

(11) **EP 4 834 368 A1**
(43) Date of publication of application: **04.11.2026**
(21) Application number: 24963786.9
(22) Date of filing: 23.12.2024
(51) Int. Cl.: A61K 45/00, A61K 33/44, A61K 33/26, A61K 9/10, A61K 47/02, A61K 47/04, A61K 31/295, A61P 35/00

(54) **USE OF IRON-LOADED CARBON NANOPARTICLE SUSPENSION INJECTION FOR COMBINED ADMINISTRATION**

(30) Priority: 18.12.2024 CN 202411873363
(71) Applicant: Sichuan Enray Pharmaceutical Technology Co., Ltd., Chengdu, Sichuan 610041 (CN)
(72) Inventor: TANG, Xiaohai, Chengdu, Sichuan 610041 (CN); HUANG, Yuanfang, Chengdu, Sichuan 610041 (CN); XIE, Ping, Chengdu, Sichuan 610041 (CN)
(74) Representative: Huang, Liwei
(86) International application number: PCT/CN2024/141520
(87) International publication number: WO 2026/129375

(57) **Abstract**

A carbon nanoparticle-Fe suspension injection for use in a method for inhibiting tumor growth, wherein the method comprises combined administration of the carbon nanoparticle-Fe suspension injection and an anticancer drug, characterised in that the anticancer drug is administered orally or intravenously, and the carbon nanoparticle-Fe suspension injection is administered through intratumoral injection. After the carbon nanoparticle-Fe suspension injection is injected into a tumor, the carbon nanoparticle-Fe suspension injection has the effect of enriching the anticancer drugs orally or intravenously administered into the tumor, improving concentrations of the anticancer drugs in the tumor, such that the anticancer drugs are enabled to act more accurately on a tumor site, doses and toxic and side effects on normal cells and tissues are relatively reduced.

## Description

### CROSS-REFERENCE TO RELATED APPLICATIONS

This application is a bypass continuation application of PCT application no.: PCT/CN2024/141520. This application claims priority from PCT Application PCT/CN2024/141520, filed December 23, 2024, and from Chinese patent application 2024118733636, filed December 18, 2024.

### TECHNICAL FIELD

The present application belongs to the field of medicine, and particularly relates to an application of carbon nanoparticle-Fe suspension injection in combined administration.

### BACKGROUND

Ferroptosis is a new iron-dependent programmed cell death mode, which is different from apoptosis, cell necrosis and autophagy. The essence of the ferroptosis lies in depletion of glutathione (GSH), decrease of glutathione peroxidase 4 (GPX4) activity, failure of lipid oxides to be metabolized by glutathione reductase catalyzed by GPX4, and then, oxidation of lipids by divalent iron ions to produce reactive oxygen species (ROS), thus promoting the ferroptosis. Main occurrence mechanisms of the ferroptosis include inhibition of cystine glutamate transport receptor (system XC-), inhibition of generation of GPX4 and ROS, inhibition of glutathione independent ferroptosis suppressor protein 1 (FSP1) and inhibition of dihydroorotate dehydrogenase (DHODH).

Carbon nanoparticle-Fe suspension injection (CNSI-Fe) is an innovative anticancer drug with carbon nanoparticles as a carrier and divalent iron ions as an effective component, which exerts an anticancer effect through a ferroptosis pathway and is used for treating pancreatic cancer, lung cancer, gastric cancer, colorectal cancer, breast cancer, cervical cancer, hepatoma, thyroid cancer, ovarian cancer, sarcoma etc. The mechanism of action is that after CNSI-Fe is locally injected into cancer tissue, and enters cancer cells through iron channels overexpressed on cancer cell membranes. Excessive iron ions enter the cancer cells rich in hydrogen peroxide (H₂O₂), and then a Fenton reaction occurs with H₂O₂ to produce a large number of hydroxyl radicals (·OH). ·OH has an extremely strong oxidation property, and reacts with unsaturated polyfatty acids (UPFAs) in the ells to produce a large number of extremely destructive lipid hydrogen peroxide (L-00H), namely lipid-reactive oxygen species (Lipid-ROS), and Lipid-ROS can destroy organelles and lead to cell destruction, triggering the ferroptosis.

Chemotherapy is the most common method in cancer treatment. Its mechanism lies in attacking normal cells and tumor cells indiscriminately, such that the chemotherapy usually causes serious toxic side effects. With development of science and technologies, targeted therapy has been found to achieve good results in some patients with few side effects. However, the targeted therapy is only applicable to some patients passing clinical screening. Sorafenib is an oral targeted therapeutic drug that blocks tumor angiogenesis, inhibits tumor cell proliferation and induces apoptosis by inhibiting multitarget kinases. Clinical trials and practical experience have shown that the sorafenib has a certain anticancer effect in some cancer treatments. Especially for advanced hepatoma and renal cell carcinoma, the sorafenib can prolong lifetime of patients and slow a rate of disease progression, and can alleviate symptoms and improve quality of life of some patients. Meanwhile, the sorafenib has been reported to exert a cancer cell killing effect by inducing a new apoptosis pathway, namely the ferroptosis. The sorafenib is not effective for all patients, its efficacy varies from individual to individual, and long-term use is prone to resistance, reducing a therapeutic effect. Moreover, the sorafenib may cause some adverse reactions, including hand-foot syndrome, rash, hypertension, diarrhea, etc. These side effects may affect the quality of life of the patients, and therefore, timely monitoring and treatment are needed.

Currently, single use of a chemotherapeutic drug or the sorafenib or CNSI-Fe requires a higher dose.

### SUMMARY

An objective of the present application is to provide a carbon nanoparticle-Fe suspension injection for use in a method for inhibiting tumor growth, so as to solve the problem that single use of a chemotherapeutic drug or sorafenib or the carbon nanoparticle-Fe suspension injection requires a higher dose.

In order to solve the above technical problem, according to some examples, a carbon nanoparticle-Fe suspension injection for use in a method for inhibiting tumor growth, wherein the method comprises combined administration, where the combined administration of the carbon nanoparticle-Fe suspension injection and anticancer drugs is used for inhibiting tumor growth, the anticancer drugs are orally or intravenously administered, and the carbon nanoparticle-Fe suspension injection is administered through intratumoral injection.

Further, the anticancer drugs are one or more selected from sorafenib, doxorubicin, cisplatin, paclitaxel, gemcitabine, docetaxel and hydroxycamptothecin.

Further, the combined administration of the carbon nanoparticle-Fe suspension injection and the anticancer drugs is for use in treating pancreatic cancer, lung cancer, gastric cancer, colorectal cancer, breast cancer, cervical cancer, hepatoma, thyroid cancer, ovarian cancer and sarcoma.

Further, the carbon nanoparticle-Fe suspension injection is prepared by mixing carbon nanoparticle suspension injection with ferrous sulfate for injection, where a concentration of the carbon nanoparticle suspension injection is 20-100 mg/mL, and a concentration of ferrous ions in the carbon nanoparticle-Fe suspension injection is 0.1-60 mg/mL.

Further, a concentration of carbon nanoparticles in the carbon nanoparticle suspension injection is 50 mg/mL, and the concentration of the ferrous ions in the carbon nanoparticle-Fe suspension injection is 7.0-60 mg/mL.

Further, the concentration of the ferrous ions in the carbon nanoparticle-Fe suspension injection is 30 mg/mL or 60 mg/mL.

Further, a particle size of the carbon nanoparticles with the ferrous ions adsorbed in the carbon nanoparticle-Fe suspension injection is 90-250 nm, and a pH value is 2.0-6.0.

Further, combined use cycles of the carbon nanoparticle-Fe suspension injection and the anticancer drugs are 1-6 cycles, and proportions within the combined use cycles are as follows:
the carbon nanoparticle-Fe suspension injection is administered once every 14 days, and 30 mg-150 mg of ferrous ions are injected each time;
proportions of the anticancer drugs include:
   the sorafenib is administered twice daily with 0.2 g-0.4 g each time; or
   the doxorubicin is administered once every three weeks with 1.2-2.4 mg/kg or 30-75 mg/m² each time; or
   the cisplatin is administered once every four weeks with 50-120 mg/m² each time, or once daily with 15-20 mg/m² each time for five consecutive days; or
   the paclitaxel is administered once weekly with 80 mg/m² each time, or once every three to four weeks with 135-175 mg/m² each time; or
   the gemcitabine is administered once weekly with 1000-1250 mg/m² each time; or
   the docetaxel is administered once every three weeks with 75 mg/m² each time; or
   the hydroxycamptothecin is administered once daily with 4-6 mg each time.

The above-mentioned technical solutions of the present invention at least have the following beneficial technical effects:
(1) After the carbon nanoparticle-Fe suspension injection is injected into a tumor, the carbon nanoparticle-Fe suspension injection has the effect of enriching the orally administered sorafenib or the intravenously administered doxorubicin, cisplatin, paclitaxel, gemcitabine, docetaxel and hydroxycamptothecin into the tumor, improving concentrations of the sorafenib, the doxorubicin, the cisplatin, the paclitaxel, the gemcitabine, the docetaxel and the hydroxycamptothecin in the tumor, such that the sorafenib, the doxorubicin, the cisplatin, the paclitaxel, the gemcitabine, the docetaxel and the hydroxycamptothecin are enabled to act more accurately on a tumor site, and doses and toxic and side effects on normal cells and tissues are relatively reduced.
(2) The sorafenib inhibits tumor proliferation by inhibiting receptor tyrosine kinase activity, the doxorubicin, the cisplatin, the paclitaxel, the gemcitabine, the docetaxel and the hydroxycamptothecin inhibit the tumor proliferation by inhibiting cell DNAs or tubulin, the carbon nanoparticle-Fe suspension injection effectively promotes ferroptosis of cancer cells by producing excessive reactive oxygen species (ROS), and after the combined administration of drugs with different mechanisms of action, a synergistic effect is achieved, thereby achieving a better anticancer effect.
(3) After administration of the carbon nanoparticle-Fe suspension injection (CNSI-Fe), the iron content in cells is increased, causing a Fenton reaction, such that hydroxyl radicals are produced, glutathione (GSH) and glutathione peroxidase 4 (GPX4) are consumed, and lipid peroxide is produced, thereby causing ferroptosis of the cells. When CNSI-Fe is administered, additional administration of the sorafenib or the cisplatin enhances cytotoxicity of the carbon nanoparticles with the ferrous ions adsorbed, such that more GSH is consumed, and more hydroxyl radicals and lipid peroxide are produced.

### BRIEF DESCRIPTION OF THE DRAWINGS

In order to more clearly illustrate technical solutions in the examples of the present application or in the prior art, a brief introduction to the accompanying drawings required for the description of the examples will be provided below. Obviously, the accompanying drawings in the following description are only some of the examples of the present application, and those of ordinary skill in the art would also be able to derive other drawings from these drawings without making creative efforts.
FIGS. 1A-1C show in-vitro isothermal adsorption curves of sorafenib adsorbed by carbon nanoparticle-Fe suspension injection with ferrous ion concentrations of 15, 30 and 60 mg/mL respectively in an example of the present application.
FIGS. 2A-2C shows in-vitro isothermal adsorption curves of doxorubicin adsorbed by carbon nanoparticle-Fe suspension injection with ferrous ion concentrations of 15, 30 and 60 mg/mL respectively in an example of the present application.
FIG. 3 is a graph comparing the iron content in cells of a control group without addition of drugs, a CNSI-Fe administration group, an SRF administration group, and a CNSI-Fe + SRF administration group in an in-vitro cell test in an example of the present application.
FIGS. 4A-4D shows graphs comparing testing results of hydroxyl radicals in cells of a control group without addition of drugs, a CNSI-Fe administration group, an SRF administration group, and a CNSI-Fe + SRF administration group in an in-vitro cell test in an example of the present application.
FIG. 5 is a graph comparing oxidative stress testing results of cells of a control group without addition of drugs, a CNSI-Fe administration group, an SRF administration group, and a CNSI-Fe + SRF administration group in an in-vitro cell test in an example of the present application.
FIG. 6 is a graph comparing GPX4 levels in cells of a control group without addition of drugs, a CNSI-Fe administration group, an SRF administration group, and a CNSI-Fe + SRF administration group in an in-vitro cell test in an example of the present application.
FIGS. 7A-7C shows graphs comparing tumor volumes of mice in a negative control group, a CNSI control group, a CDDP control group, a CNSI-Fe administration group, an SRF administration group, and a CNSI-Fe + SRF administration group of a 4T1 breast cancer model, an H22 hepatoma model, and an AsPC-1 pancreatic cancer model in an in-vivo test in an example of the present application.
FIGS. 8A-8D shows graphs comparing hydroxyl radicals of mouse tumors in a negative control group, a CNSI-Fe administration group, an SRF administration group, and a CNSI-Fe + SRF administration group in an in-vivo test in an example of the present application.
FIGS. 9A-9C shows graphs comparing the SRF content in tumor tissues of mice in an SRF administration group and a CNSI-Fe + SRF administration group of a 4T1 breast cancer model, an H22 hepatoma model, and AsPC-1 pancreatic cancer model in an in-vivo test in an example of the present application.
FIG. 10 is a graph comparing the SRF content in tumor cells of mice in an SRF administration group and a CNSI-Fe + SRF administration group in an in-vivo test in an example of the present application.
FIGS. 11A-11B shows change graphs comparing tumor volumes of subcutaneous tumors of CT26.WT colon cancer and subcutaneous tumors of HT1080 fibrosarcoma after treatment of a negative control group, a CNSI-Fe group, a DOX group and a CNSI-Fe + DOX group in an in-vivo test in an example of the present application.
FIGS. 12A-12B shows concentrations of DOX in tumor tissues of subcutaneous tumors of CT26.WT colon cancer and subcutaneous tumors of HT1080 fibrosarcoma of a DOX group and a CNSI-Fe + DOX group in an in-vivo test in an example of the present application.
FIG. 13 is a change graph comparing tumor volumes of subcutaneous tumors of hepatoma after treatment of a negative control group, a CNSI-Fe group, a CDDP group, and a CNSI-Fe + CDDP group in an in-vivo test in an example of the present application.
FIG. 14 shows concentrations of CDDP in tumor tissues of subcutaneous tumors of hepatoma of a CDDP group and a CNSI-Fe + CDDP group in an in-vivo test in an example of the present application.
FIG. 15 is a change graph comparing tumor volumes of subcutaneous tumors of breast cancer after treatment of a negative control group, a CNSI-Fe group, a PTX group and a CNSI-Fe + PTX group in an in-vivo test in an example of the present application.
FIG. 16 shows concentrations of PTX in tumor tissues of subcutaneous tumors of breast cancer of a PTX group and a CNSI-Fe + PTX group in an in-vivo test in an example of the present application.
FIG. 17 is a change graph comparing tumor volumes of subcutaneous tumors of pancreatic cancer after treatment of a negative control group, a CNSI-Fe group, a GEM group and a CNSI-Fe + GEM group in an in-vivo test in an example of the present application.
FIG. 18 shows concentrations of GEM in tumor tissues of subcutaneous tumors of pancreatic cancer of a GEM group and a CNSI-Fe + GEM group in an in-vivo test in an example of the present application.
FIG. 19 is a change graph comparing tumor volumes of subcutaneous tumors of breast cancer after treatment of a negative control group, a CNSI-Fe group, a DTX group and a CNSI-Fe + DTX group in an in-vivo test in an example of the present application.
FIG. 20 shows concentrations of DTX in tumor tissues of subcutaneous tumors of breast cancer in a DTX group and a CNSI-Fe + DTX group in an in-vivo test in an example of the present application.
FIG. 21 is a change graph comparing tumor volumes of subcutaneous tumors of colon cancer after treatment of a negative control group, a CNSI-Fe group, a HCPT group and a CNSI-Fe + HCPT group in an in-vivo test in an example of the present application.
FIG. 22 shows concentrations of HCPT in tumor tissues of subcutaneous tumors of colon cancer in a HCPT group and a CNSI-Fe + HCPT group in an in-vivo test in an example of the present application.
FIG. 23 is a change graph comparing tumor volumes of subcutaneous tumors of pancreatic cancer after treatment of a negative control group, a CNSI-Fe group, a PTX + GEM group, and a CNSI-Fe + PTX + GEM group in an in-vivo test in an example of the present application.

### DETAILED DESCRIPTIONS OF THE EMBODIMENTS

In order to make objectives, technical solutions and advantages of the examples of the present application clearer, the following will describe the examples of the present application in detail with reference to the accompanying drawings. However, those of ordinary skill in the art may understand that in each example of the present application, many technical details have been put forward in order to make readers better understand the present application. However, even without these technical details and various changes and modifications based on the following examples, technical solutions to be protected as required in the claims of the present application may be realized. The following examples are divided for convenience of description, and should not constitute any limitation on the specific implementation mode of the present application. The examples may be combined with each other and referred to each other without contradiction.

Currently, the problem that single use of an anticancer drug or the carbon nanoparticle-Fe suspension injection (CNSI-Fe) requires a higher dose exists in the prior art.

In order to solve the above problem, an example of the present application provides an application of carbon nanoparticle-Fe suspension injection in combined administration, where the combined administration of the carbon nanoparticle-Fe suspension injection and one or more selected from sorafenib (SRF), doxorubicin (DOX), cisplatin (CDDP), paclitaxel (PTX), gemcitabine (GEM), docetaxel (DTX) and hydroxycamptothecin (HCPT) may be used for treating cancer and mainly used for treating pancreatic cancer, lung cancer, gastric cancer, colorectal cancer, breast cancer, cervical cancer, hepatoma, thyroid cancer, ovarian cancer and sarcoma. The sorafenib is orally administered, the doxorubicin, cisplatin, paclitaxel, gemcitabine, docetaxel and hydroxycamptothecin are intravenously administered, and the carbon nanoparticle-Fe suspension injection is administered through intratumoral injection. The carbon nanoparticle-Fe suspension injection is prepared by mixing carbon nanoparticle suspension injection and ferrous sulfate for injection.

Combined administration is a common strategy for clinical treatment of cancer. The combined administration is more likely to improve a therapeutic effect, reduce risk of resistance, and reduce side effects that patients face. The advantage of this kind of comprehensive treatment is that a complementary effect of different drugs or treatment methods can be utilized, and aiming at multiple growth and development ways of tumors, the comprehensive treatment can inhibit growth and diffusion of the tumors more comprehensively, thereby improving a comprehensive effect of the treatment.

It is found through research of the present application that, after the carbon nanoparticle-Fe suspension injection is injected into the tumor, the carbon nanoparticle-Fe suspension injection has the effect of enriching the orally administered sorafenib or the intravenously administered doxorubicin, cisplatin, paclitaxel, gemcitabine, docetaxel and hydroxycamptothecin into the tumor, improving concentrations of the sorafenib, the doxorubicin, the cisplatin, the paclitaxel, the gemcitabine, the docetaxel and the hydroxycamptothecin in the tumor, such that the sorafenib, the doxorubicin, the cisplatin, the paclitaxel, the gemcitabine, the docetaxel and the hydroxycamptothecin are enabled to act more accurately on a tumor site, and doses and toxic and side effects on normal cells and tissues are relatively reduced.

In addition, the carbon nanoparticle-Fe suspension injection has an anticancer effect, and exerts an anticancer effect through a ferroptosis pathway. The carbon nanoparticle-Fe suspension injection is a nanosuspension anticancer drug which takes carbon nanoparticles as a carrier and ferrous ions as an effective component, directly causes targeted ferroptosis of Fe²⁺ through intratumoral injection, and can effectively inhibit tumor growth (CNSI-Fe²⁺+H2O2→ROS(·OH)→L-ROS→Ferroptosis). The sorafenib is an oral multikinase inhibitor, which can directly inhibit tumor growth by inhibiting a Raf/MEK/ERK signal transduction pathway, can also indirectly inhibit tumor growth by inhibiting activity of a tyrosine kinase receptor related to angiogenesis and tumor growth and blocking angiogenesis of the tumor, and can also inhibit tumor growth by inhibiting cystine/glutamate antiporter (system xc-), glutathione (GSH) depletion and iron-dependent accumulation of lipid reactive oxygen species (ROS), causing ferroptosis. The doxorubicin is a broad-spectrum anticancer drug that exerts an effect primarily by being inserted into cellular DNAs and triggering topoisomerase II to destroy a tertiary structure of the DNAs. The cisplatin inhibits cell proliferation mainly by being bond to the DNAs to form Pt/DNA adducts, which destroys the structure of the DNAs, and can also induce ferroptosis of cells by consuming GSH, inactivating glutathione peroxidase (GPX), disrupting iron metabolism, producing ROS and promoting lipid peroxidation. The paclitaxel mainly acts on tubulin. By stabilizing and enhancing polymerization of the tubulin, the paclitaxel prevents microtubule depolymerization, breaks normal dynamic balance of microtubule polymerization and depolymerization, causes the cells to fail to form spindles and spindle filaments during mitosis, inhibits cell division and proliferation, and thus exerts an antitumor effect. The gemcitabine is a pyrimidine nucleotide analog that mainly acts in a DNA synthesis phase(namely phase S), and is activated by deoxycytosine kinase to form gemcitabine diphosphate (dFdCDP) and gemcitabine triphosphate (dFdCTP), which can inhibit DNA synthesis and lead to apoptosis. The docetaxel is a taxol antitumor drug which can be bound to free tubulin, promotes assembly of tubulin into stable microtubules, and inhibits depolymerization of the tubulin, resulting in generation of microtubule bundles that lose a normal function and fixation of the microtubules, thereby inhibiting mitosis of the cells. The hydroxycamptothecin is an inhibitor of DNA synthesis, which mainly acts on a DNA synthesis phase (i.e. phase S), inhibits DNA topoisomerase I, and obviously inhibits nucleic acid synthesis, especially the DNA synthesis. These drugs with different mechanisms of action can block different signal pathways, change a tumor microenvironment, overcome tumor heterogeneity, and have a synergistic effect when being combined with the carbon nanoparticle-Fe suspension injection, thus playing a better anticancer effect, and resistance may also be reduced.

In addition, after administration of the carbon nanoparticle-Fe suspension injection, the iron content in the cells is increased, causing a Fenton reaction, such that hydroxyl radicals are produced, GSH and GPX4 are consumed, and lipid peroxide is produced, thereby causing ferroptosis of the cells. When the carbon nanoparticle-Fe suspension injection is administered, additional administration of the sorafenib or the cisplatin enhances cytotoxicity of the carbon nanoparticles with the ferrous ions adsorbed, such that more GSH is consumed, and more hydroxyl radicals and lipid peroxide are produced.

In an example of the present application, in the course of the combined administration, the sorafenib is administered orally or directly by gavage, followed by intratumoral injection of the carbon nanoparticle-Fe suspension injection, or intratumoral injection of the carbon nanoparticle-Fe suspension injection followed by intravenous injection of chemotherapeutic drugs. The optional process is as follows:
The carbon nanoparticle-Fe suspension injection is administered once every 14 days, and 30 mg-150 mg of ferrous ions are injected each time;
proportions of the anticancer drugs include: the sorafenib is administered twice daily with 0.2 g-0.4 g each time;
or, the doxorubicin is administered once every three weeks with 1.2-2.4 mg/kg or 30-75 mg/m² each time; or
the cisplatin is administered once every four weeks with 50-120 mg/m² each time, or once daily with 15-20 mg/m² each time for five consecutive days; or
the paclitaxel is administered once weekly with 80 mg/m² each time, or once every three to four weeks with 135-175 mg/m² each time; or
the gemcitabine is administered once weekly with 1000-1250 mg/m² each time; or
the docetaxel is administered once every three weeks with 75 mg/m² each time; or
the hydroxycamptothecin is administered once daily with 4-6 mg each time.

In the above units of measurement, mg/m² represents drug weight/body surface area, and mg/kg represents drug weight/body weight. In an example of the present application, the carbon nanoparticle-Fe suspension injection is prepared by mixing carbon nanoparticle suspension injection and ferrous sulfate for injection.

During storage, the carbon nanoparticle suspension injection and the ferrous sulfate for injection are stored separately. During clinical use, the ferrous sulfate for injection is dissolved in the carbon nanoparticle suspension injection to form the carbon nanoparticle-Fe suspension injection, where a concentration of carbon nanoparticles in the carbon nanoparticle suspension injection is 20-100 mg/mL, and a concentration of ferrous ions in the carbon nanoparticle-Fe suspension injection is 0.1-60 mg/mL. Preferably, a concentration of carbon nanoparticles in the carbon nanoparticle suspension injection is 50 mg/mL, and the concentration of the ferrous ions in the carbon nanoparticle-Fe suspension injection is 7.0-60 mg/mL. More preferably, the concentration of the ferrous ions in the carbon nanoparticle-Fe suspension injection is 30 mg/mL or 60 mg/mL.

In an example of the present application, a particle size of the carbon nanoparticles with the ferrous ions adsorbed is 90-250 nm, and a pH value is 2.0-6.0. The carbon nanoparticle-Fe suspension injection enters the cells through an iron input channel overexpressed on cancer cell membranes or phagocytosis, and enables the sorafenib, the doxorubicin, the cisplatin, the paclitaxel, the gemcitabine, the docetaxel or the hydroxycamptothecin to be enriched.

In an example of the present application, a preparation process for the carbon nanoparticle suspension injection is as follows:
60 mg of sodium citrate and 400 mg of poloxamer are weighed and dissolved in 20 mL of normal saline. Then, 1000 mg of bulk drugs of carbon nanoparticles are added. Homogenization is performed with a homogenizer for 5-10 minutes at a rotation speed of 7000 rpm. The product is transferred to a fine homogenizer for fine homogenization three times after completion of homogenization (pressure of fine homogenization being 20000 psi) to obtain the carbon nanoparticle suspension injection. Optionally, the bulk drugs of carbon nanoparticles is carbon black (C₄₀).

A preparation method for the ferrous sulfate for injection is as follows:
1490 mg of bulk drugs of ferrous sulfate heptahydrate is dissolved in 20 mL of water for injection, and pH of the solution is adjusted with sulfuric acid to 2.8 after completion of dissolution.

Then, packaging and freeze-drying are performed, nitrogen is backwashed, and lips are pressed, thereby obtaining the ferrous sulfate for injection.

When the carbon nanoparticle-Fe suspension injection needs to be used, a preparation method for the carbon nanoparticle-Fe suspension injection is as follows: the carbon nanoparticle suspension injection is sucked and added into the ferrous sulfate for injection (bottled) under the condition of isolating air, and uniformly mixed.

A preparation method for the carbon nanoparticle-Fe suspension injection with different ferrous ion concentrations is as follows:
In the case that the concentration of the ferrous ions is 60 mg/mL, 0.5 mL of carbon nanoparticle suspension injection is taken with a syringe, and the syringe passes through a rubber stopper of a ferrous sulfate bottle for injection under the condition of isolating air. The carbon nanoparticle suspension injection is injected into the ferrous sulfate for injection to be mixed, and shaken well. After mixing for 5 minutes, the mixture is shaken well to obtain the required carbon nanoparticle-Fe suspension injection.

In the case that the concentration of the ferrous ions is 30 mg/mL, 1 mL of carbon nanoparticle suspension injection is taken with a syringe, and the syringe passes through a rubber stopper of a ferrous sulfate bottle for injection under the condition of isolating air. The carbon nanoparticle suspension injection is injected into the ferrous sulfate for injection to be mixed, and shaken well. After mixing for 5 minutes, the mixture is shaken well to obtain the required carbon nanoparticle-Fe suspension injection.

In the case that the concentration of the ferrous ions is 15 mg/mL, 2 mL of carbon nanoparticle suspension injection is taken with a syringe, and the syringe passes through a rubber stopper of a ferrous sulfate bottle for injection under the condition of isolating air. The carbon nanoparticle suspension injection is injected into the ferrous sulfate for injection to be mixed, and shaken well. After mixing for 5 minutes, the mixture is shaken well to obtain the required carbon nanoparticle-Fe suspension injection.

In the case that the concentration of the ferrous ions is 7.5 mg/mL, 4 mL of carbon nanoparticle suspension injection is taken with a syringe, and the syringe passes through a rubber stopper of a ferrous sulfate bottle for injection under the condition of isolating air. The carbon nanoparticle suspension injection is injected into the ferrous sulfate for injection to be mixed, and shaken well. After mixing for 5 minutes, the mixture is shaken well to obtain the required carbon nanoparticle-Fe suspension injection.

In the case that the concentration of the ferrous ions is 3.75 mg/mL, 8 mL of carbon nanoparticle suspension injection is taken with a syringe, and the syringe passes through a rubber stopper of a ferrous sulfate bottle for injection under the condition of isolating air. The carbon nanoparticle suspension injection is injected into the ferrous sulfate for injection to be mixed, and shaken well. After mixing for 5 minutes, the mixture is shaken well to obtain the required carbon nanoparticle-Fe suspension injection.

The following description is combined with specific tests, and the tests are divided into an in-vitro isothermal adsorption test, an in-vitro cell test and an in-vivo test:

The in-vitro isothermal adsorption test is as follows:

### Example 1

Sorafenib solutions (mg/mL) were prepared in a series of concentrations, 1 mL of carbon nanoparticle-Fe suspension injection (a concentration of carbon nanoparticle suspension injection being 50 mg/mL, and concentrations of ferrous ions being 15, 30 and 60 mg/mL respectively), 2.8 mL of methanol, and 0.2 mL of a sorafenib solution were added sequentially to a 10 mL centrifuge tube, mixed well, then shaken (110 r/min) at a shaking table at 37°C for 1 h, and centrifuged at 8000 r/min for 10 min. 0.2 mL of supernatant was taken and mixed well with 1 mL of methanol. The concentration of sorafenib was tested by high performance liquid chromatography (HPLC) through membranes, and adsorption capacity (Qe) was calculated according to a Langmuir model.

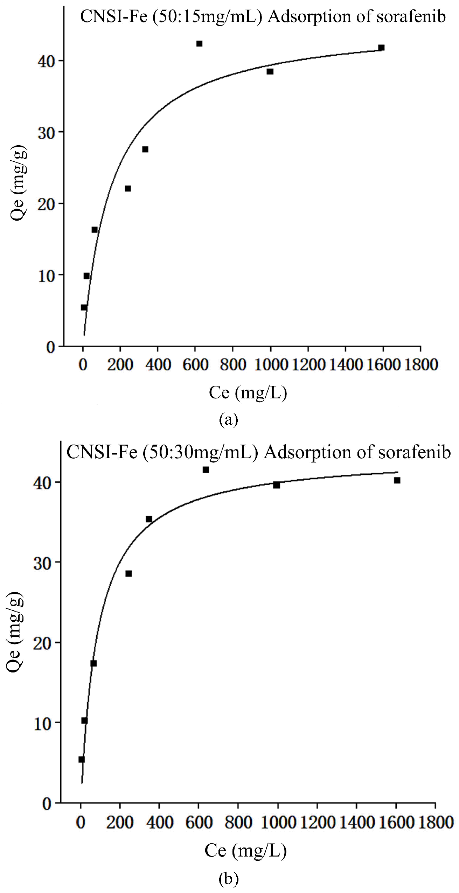

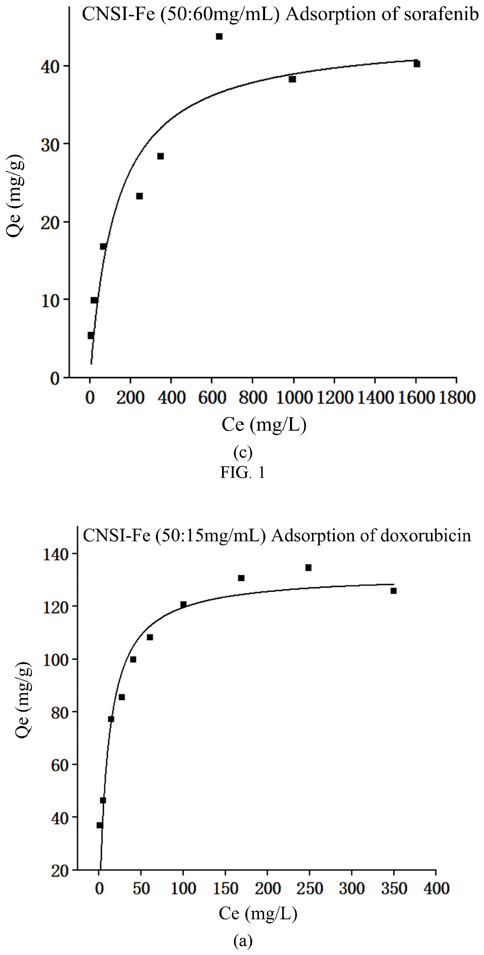

Testing results show that the maximum adsorption capacity of the sorafenib by the carbon nanoparticle-Fe suspension injection with ferrous ion concentrations of 15 mg/mL, 30 mg/mL and 60 mg/mL is similar, which is 42.36 mg/g, 41.57 mg/g and 43.79 mg/g respectively. Isothermal adsorption curves are shown in FIGS. 1A-1C, where FIG. 1A is an isothermal adsorption curve of the sorafenib by the carbon nanoparticle-Fe suspension injection with the ferrous ion concentration of 15 mg/mL, FIG. 1B is an isothermal adsorption curve of the sorafenib by the carbon nanoparticle-Fe suspension injection with the ferrous ion concentration of 30 mg/mL, and FIG. 1C is an isothermal adsorption curve of the sorafenib by the carbon nanoparticle-Fe suspension injection with the ferrous ion concentration of 60 mg/mL.

### Example 2

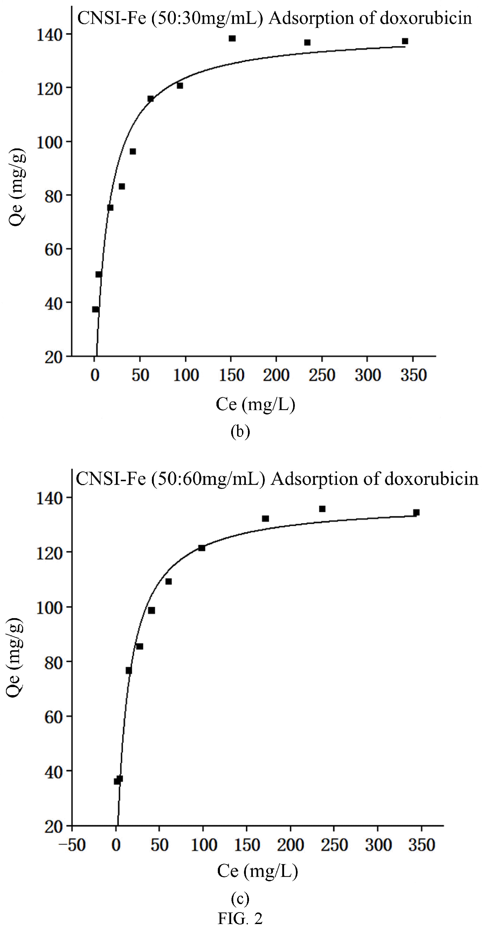

Individual doxorubicin solutions (mg/mL) were prepared in a series of concentrations, and isothermal adsorption tests were performed in the same manner as in Example 1. Results show that the maximum adsorption capacity of the doxorubicin by the carbon nanoparticle-Fe suspension injection with ferrous ion concentrations of 15 mg/mL, 30 mg/mL and 60 mg/mL is similar, which is 134.57 mg/g, 138.37 mg/g and 135.79 mg/g respectively. Isothermal adsorption curves are shown in FIGS. 2A-2C, where FIG. 2A is an isothermal adsorption curve of the doxorubicin by the carbon nanoparticle-Fe suspension injection with the ferrous ion concentration of 15 mg/mL, FIG. 2B is an isothermal adsorption curve of the doxorubicin by the carbon nanoparticle-Fe suspension injection with the ferrous ion concentration of 30 mg/mL, and FIG. 2C is an isothermal adsorption curve of the doxorubicin by the carbon nanoparticle-Fe suspension injection with the ferrous ion concentration of 60 mg/mL.

### Example 3

Individual cisplatin solutions (mg/mL) were prepared in a series of concentrations, and isothermal adsorption tests were performed in the same manner as in Example 1. Results show that the maximum adsorption capacity of the cisplatin by the carbon nanoparticle-Fe suspension injection with the ferrous ion concentration of 15 mg/mL is 8.20 mg/g.

### Example 4

Individual paclitaxel solutions (mg/mL) were prepared in a series of concentrations, and isothermal adsorption tests were performed in the same manner as in Example 1. Results show that the maximum adsorption capacity of the paclitaxel by the carbon nanoparticle-Fe suspension injection with the ferrous ion concentration of 15 mg/mL is 35.98 mg/g.

### Example 5

Individual gemcitabine solutions (mg/mL) were prepared in a series of concentrations, and isothermal adsorption tests were performed in the same manner as in Example 1. Results show that the maximum adsorption capacity of the gemcitabine by the carbon nanoparticle-Fe suspension injection with the ferrous ion concentration of 15 mg/mL is 19.23 mg/g.

### Example 6

Individual docetaxel solutions (mg/mL) were prepared in a series of concentrations, and isothermal adsorption tests were performed in the same manner as in Example 1. Results show that the maximum adsorption capacity of the docetaxel by the carbon nanoparticle-Fe suspension injection with the ferrous ion concentration of 15 mg/mL is 46.77 mg/g.

### Example 7

Individual hydroxycamptothecin solutions (mg/mL) were prepared in a series of concentrations, and isothermal adsorption tests were performed in the same manner as in Example 1. Results show that the maximum adsorption capacity of the hydroxycamptothecin by the carbon nanoparticle-Fe suspension injection with the ferrous ion concentration of 15 mg/mL is 12.41 mg/g.

The in-vitro cell test is as follows:

### Example 8

Test materials: RPMI-1640 medium, dulbecco's modified eagle medium (DMEM), fetal bovine serum (FBS), cell digestion solution trypsin, penicillin streptomycin mixture, phosphate buffer solution (PBS, pH 7.4), carbon nanoparticle suspension injection, and carbon nanoparticle-Fe suspension injection (carbon nanoparticle-ferrous sulfate, CNSI-Fe, concentration being measured in ferrous ions), sorafenib (SRF), doxorubicin (DOX), cisplatin (CDDP), paclitaxel (PTX), gemcitabine (GEM), docetaxel (DTX) and hydroxycamptothecin (HCPT).

Cell lines: murine 4T1 triple negative breast cancer cells, murine CT26.WT colon cancer cells, murine H22 hepatoma cells, and human AsPC-1 pancreatic cancer cells.

Test method: Cancer cells growing in a logarithmic phase were collected and cultured in a 12-well plate after a concentration of the cell suspension was adjusted. Incubation was performed at 37°C for 24 h in 5% CO₂. An experiment was divided into 5 groups: a control group without addition of drugs, a CNSI group, a CNSI-Fe administration group, a series concentration anticancer drug administration group and a CNSI-Fe + series concentration anticancer drug administration group, where three replicate holes were made for each drug concentration of each group.
(1) A complete medium containing serial concentrations of anticancer drugs was added to the serial concentration anticancer drug administration group.
(2) A complete medium containing serial concentrations of anticancer drugs was added to the CNSI-Fe + series concentration anticancer drug administration group for culture for 6 h, and then a CNSI-Fe solution was added to make the final concentration of Fe ions reach 100 µg/mL.
(3) A complete medium containing 333 µg/mL of carbon nanoparticles was added to the CNSI group.
(4) A complete medium with an iron ion concentration of 100 µg/mL was added to the CNSI-Fe administration group.
(5) No drugs were added to the control group without addition of drugs.

Then, the above (1)-(5) groups were incubated in 5% CO₂ at 37°C for 48 h.

The medium was discarded, and the cells were collected. The number of cells was counted under an optical microscope, an inhibition ratio is calculated, and a q value was calculated according to the formula q=E_{A+B}/(E_{A}+E_{B}-E_{A}×E_{B}) for a combined effect (q), where E_{A} represented an effect of drug A when administered alone, E_{B} represented an effect of drug B when administered alone, E_{A+B} represented an effect of drug A and B when administered together, which was an expected value of the combined effect of the two drugs. If an actual combined effect obtained is equal to the expected value, that is, q=1 represents being additive, q>1 represents being synergistic, and q<1 represents being antagonistic. testing results are shown in Tables 1-7.

### 1. Combined administration of carbon nanoparticle-Fe suspension injection (CNSI-Fe) and sorafenib (SRF) in vitro:

**Table 1: Inhibition rate (%) and q value of CNSI, CNSI-Fe, SRF, CNSI-Fe + SRF against 4T1 triple negative breast cancer in in-vitro cell test**

| Concentration (µg/mL) | Inhibition rate (%) | q value of combined effect | Synergistic effect or not |
|---|---|---|---|
| CNSI 333 | 3.55±2.88 | NA | NA |
| CNSI-Fe 100 | 46.34±1.94 | NA | NA |
| SRF 4 | 84.01±1.41 | NA | NA |
| SRF 2 | 47.32±1.56 | NA | NA |
| SRF 1 | 38.45±2.08 | NA | NA |
| SRF 0.5 | 36.20±3.46 | NA | NA |
| SRF 0.25 | 22.25±7.56 | NA | NA |
| SRF 0.125 | 20.77±5.42 | NA | NA |
| CNSI-Fe 100 + SRF 4 | 84.22±2.52 | 0.92 | No |
| CNSI-Fe 100 + SRF 2 | 80.35±2.21 | 1.12 | Yes |
| CNSI-Fe 100 + SRF 1 | 78.80±3.18 | 1.18 | Yes |
| CNSI-Fe 100 + SRF 0.5 | 72.82±3.06 | 1.11 | Yes |
| CNSI-Fe 100 + SRF 0.25 | 71.55±2.14 | 1.28 | Yes |
| CNSI-Fe 100 + SRF 0.125 | 63.38±7.10 | 1.10 | Yes |

As can be seen from testing results in Table 1:
Administration of carbon nanoparticles alone has no effect on growth of cancer cells, and the inhibition rate is 3.55±2.88%.

Administration of carbon nanoparticle-Fe suspension injection alone has a significant inhibition effect on cell growth, and the inhibition rate is 46.34±1.94%.

After combined administration of the carbon nanoparticle-Fe suspension injection and sorafenib at different concentrations, q values are all >1 when the concentration of the sorafenib is ≤2 µg/mL, which shows a synergistic effect on cell growth inhibition (Table 1), indicating that the combined administration of the carbon nanoparticle-Fe suspension injection and the sorafenib can enhance cytotoxicity of the carbon nanoparticles with the ferrous ions adsorbed.

### 2. Combined administration of carbon nanoparticle-Fe suspension injection (CNSI-Fe) and doxorubicin (DOX) in vitro:

**Table 2: Inhibition rate (%) and q value of CNSI, CNSI-Fe, DOX, CNSI-Fe + DOX against CT26.WT colon cancer in in-vitro cell test**

| Concentration (µg/mL) | Inhibition rate (%) | q value of combined effect | Synergistic effect or not |
|---|---|---|---|
| CNSI 333 | 4.67±2.38 | NA | NA |
| CNSI-Fe 100 | 53.47±2.09 | NA | NA |
| DOX 4 | 79.85±1.96 | NA | NA |
| DOX 2 | 64.59±2.31 | NA | NA |
| DOX 1 | 50.24±2.19 | NA | NA |
| DOX 0.5 | 39.57±3.01 | NA | NA |
| DOX 0.25 | 24.39±4.15 | NA | NA |
| DOX 0.125 | 19.57±4.91 | NA | NA |
| CNSI-Fe 100 + DOX 4 | 87.59±3.57 | 0.97 | No |
| CNSI-Fe 100 + DOX 2 | 85.98±3.78 | 1.03 | Yes |
| CNSI-Fe 100 + DOX 1 | 80.19±1.47 | 1.04 | Yes |
| CNSI-Fe 100 + DOX 0.5 | 76.39±3.78 | 1.06 | Yes |
| CNSI-Fe 100 + DOX 0.25 | 75.48±4.09 | 1.16 | Yes |
| CNSI-Fe 100 + DOX 0.125 | 70.79±3.70 | 1.13 | Yes |

As can be seen from testing results in Table 2:
Administration of carbon nanoparticles alone has no effect on growth of cancer cells, and the inhibition rate is 4.67±2.38%.

Administration of carbon nanoparticle-Fe suspension injection alone has a significant inhibition effect on cell growth, and the inhibition rate is 53.47±2.09%.

After combined administration of the carbon nanoparticle-Fe suspension injection and doxorubicin at different concentrations, q values are all >1 when the concentration of the doxorubicin is ≤2 µg/mL, which shows a synergistic effect on cell growth inhibition (Table 2), indicating that the combined administration of the carbon nanoparticle-Fe suspension injection and the doxorubicin can enhance cytotoxicity of the carbon nanoparticles with the ferrous ions adsorbed.

### 3. Combined administration of carbon nanoparticle-Fe suspension injection (CNSI-Fe) and cisplatin (CDDP) in vitro:

**Table 3: Inhibition rate (%) and q value of CNSI, CNSI-Fe, CDDP, CNSI-Fe + CDDP against H22 hepatoma in in-vitro cell test**

| Concentration (µg/mL) | Inhibition rate (%) | q value of combined effect | Synergistic effect or not |
|---|---|---|---|
| CNSI 333 | 5.18±3.42 | NA | NA |
| CNSI-Fe 100 | 55.34±3.79 | NA | NA |
| CDDP 20 | 86.47±4.08 | NA | NA |
| CDDP 10 | 73.24±3.72 | NA | NA |
| CDDP 5 | 57.41±4.79 | NA | NA |
| CDDP 2.5 | 42.93±3.56 | NA | NA |
| CDDP 1.25 | 34.29±3.95 | NA | NA |
| CDDP 0.63 | 26.47±4.09 | NA | NA |
| CNSI-Fe 100 + CDDP 20 | 95.27±4.12 | 1.01 | Yes |
| CNSI-Fe 100 + CDDP 10 | 90.56±3.47 | 1.03 | Yes |
| CNSI-Fe 100 + CDDP 5 | 85.79±2.19 | 1.06 | Yes |
| CNSI-Fe 100 + CDDP 2.5 | 83.47±1.65 | 1.12 | Yes |
| CNSI-Fe 100 + CDDP 1.25 | 81.28±1.37 | 1.15 | Yes |
| CNSI-Fe 100 + CDDP 0.63 | 77.16±2.85 | 1.15 | Yes |

As can be seen from testing results in Table 3:
Administration of carbon nanoparticles alone has no effect on growth of cancer cells, and the inhibition rate is 5.18±3.42%.

Administration of carbon nanoparticle-Fe suspension injection alone has a significant inhibition effect on cell growth, and the inhibition rate is 55.34±3.79%.

After combined administration of the carbon nanoparticle-Fe suspension injection and cisplatin at different concentrations, q values are all >1 when the concentration of the cisplatin is ≤20 µg/mL, which shows a synergistic effect on cell growth inhibition (Table 3), indicating that the combined administration of the carbon nanoparticle-Fe suspension injection and the cisplatin can enhance cytotoxicity of the carbon nanoparticles with the ferrous ions adsorbed.

### 4. Combined administration of carbon nanoparticle-Fe suspension injection (CNSI-Fe) and paclitaxel (PTX) in vitro:

**Table 4: Inhibition rate (%) and q value of CNSI, CNSI-Fe, PTX, CNSI-Fe + PTX against 4T1 breast cancer in in-vitro cell test**

| Concentration (µg/mL) | Inhibition rate (%) | q value of combined effect | Synergistic effect or not |
|---|---|---|---|
| CNSI 333 | 3.05±1.69 | NA | NA |
| CNSI-Fe 100 | 54.39±1.29 | NA | NA |
| PTX 1 | 88.24±3.25 | NA | NA |
| PTX 0.5 | 70.16±2.48 | NA | NA |
| PTX 0.25 | 53.12±1.98 | NA | NA |
| PTX 0.125 | 44.36±3.32 | NA | NA |
| PTX 0.063 | 30.15±3.15 | NA | NA |
| PTX 0.032 | 22.10±2.89 | NA | NA |
| CNSI-Fe 100 + PTX 1 | 96.13±2.69 | 1.02 | Yes |
| CNSI-Fe 100 + PTX 0.5 | 91.39±3.07 | 1.06 | Yes |
| CNSI-Fe 100 + PTX 0.25 | 88.69±3.24 | 1.13 | Yes |
| CNSI-Fe 100 + PTX 0.125 | 84.16±4.98 | 1.13 | Yes |
| CNSI-Fe 100 + PTX 0.063 | 80.79±3.14 | 1.19 | Yes |
| CNSI-Fe 100 + PTX 0.032 | 74.17±1.78 | 1.15 | Yes |

As can be seen from testing results in Table 4:
Administration of carbon nanoparticles alone has no effect on growth of cancer cells, and the inhibition rate is 3.05±1.69%.

Administration of carbon nanoparticle-Fe suspension injection alone has a significant inhibition effect on cell growth, and the inhibition rate is 54.39±1.29%.

After combined administration of the carbon nanoparticle-Fe suspension injection and paclitaxel at different concentrations, q values are all >1 when the concentration of the paclitaxel is ≤1 µg/mL, which shows a synergistic effect on cell growth inhibition (Table 4), indicating that the combined administration of the carbon nanoparticle-Fe suspension injection and the paclitaxel can enhance cytotoxicity of the carbon nanoparticles with the ferrous ions adsorbed.

### 5. Combined administration of carbon nanoparticle-Fe suspension injection (CNSI-Fe) and gemcitabine (GEM) in vitro:

**Table 5: Inhibition rate (%) and q value of CNSI, CNSI-Fe, GEM, CNSI-Fe + GEM against AsPC-1 pancreatic cancer in in-vitro cell test**

| Concentration (µg/mL) | Inhibition rate (%) | q value of combined effect | Synergistic effect or not |
|---|---|---|---|
| CNSI 333 | 3.77±2.55 | NA | NA |
| CNSI-Fe 100 | 50.74±2.97 | NA | NA |
| GEM 0.08 | 85.16±4.03 | NA | NA |
| GEM 0.04 | 78.56±3.24 | NA | NA |
| GEM 0.02 | 70.12±3.37 | NA | NA |
| GEM 0.01 | 53.84±3.10 | NA | NA |
| GEM 0.005 | 32.15±1.34 | NA | NA |
| GEM 0.003 | 19.73±1.78 | NA | NA |
| CNSI-Fe 100 + GEM 0.08 | 93.25±3.28 | 1.01 | Yes |
| CNSI-Fe 100 + GEM 0.04 | 90.47±3.01 | 1.01 | Yes |
| CNSI-Fe 100 + GEM 0.02 | 86.28±3.47 | 1.01 | Yes |
| CNSI-Fe 100 + GEM 0.01 | 81.97±2.74 | 1.06 | Yes |
| CNSI-Fe 100 + GEM 0.005 | 79.67±2.22 | 1.20 | Yes |
| CNSI-Fe 100 + GEM 0.003 | 71.05±2.07 | 1.18 | Yes |

As can be seen from testing results in Table 5:
Administration of carbon nanoparticles alone has no effect on growth of cancer cells, and the inhibition rate is 3.77±2.55%.

Administration of carbon nanoparticle-Fe suspension injection alone has a significant inhibition effect on cell growth, and the inhibition rate is 50.74±2.97%.

After combined administration of the carbon nanoparticle-Fe suspension injection and gemcitabine at different concentrations, q values are all >1 when the concentration of the gemcitabine is ≤0.08 µg/mL, which shows a synergistic effect on cell growth inhibition (Table 5), indicating that the combined administration of the carbon nanoparticle-Fe suspension injection and the gemcitabine can enhance cytotoxicity of the carbon nanoparticles with the ferrous ions adsorbed.

### 6. Combined administration of carbon nanoparticle-Fe suspension injection (CNSI-Fe) and docetaxel (DTX) in vitro:

**Table 6: Inhibition rate (%) and q value of CNSI, CNSI-Fe, DTX, CNSI-Fe + DTX against 4T1 breast cancer in in-vitro cell test**

| Concentration (µg/mL) | Inhibition rate (%) | q value of combined effect | Synergistic effect or not |
|---|---|---|---|
| CNSI 333 | 5.35±3.14 | NA | NA |
| CNSI-Fe 100 | 49.87±2.97 | NA | NA |
| DTX 1 | 82.69±3.02 | NA | NA |
| DTX 0.5 | 71.24±4.75 | NA | NA |
| DTX 0.25 | 50.12±2.97 | NA | NA |
| DTX 0.125 | 38.47±3.47 | NA | NA |
| DTX 0.063 | 31.24±3.39 | NA | NA |
| DTX 0.032 | 17.57±2.14 | NA | NA |
| CNSI-Fe 100 + DTX 1 | 92.12±2.69 | 1.01 | Yes |
| CNSI-Fe 100 + DTX 0.5 | 86.17±3.07 | 1.01 | Yes |
| CNSI-Fe 100 + DTX 0.25 | 85.47±3.24 | 1.14 | Yes |
| CNSI-Fe 100 + DTX 0.125 | 80.36±4.98 | 1.16 | Yes |
| CNSI-Fe 100 + DTX 0.063 | 71.20±3.14 | 1.09 | Yes |
| CNSI-Fe 100 + DTX 0.032 | 69.87±1.78 | 1.19 | Yes |

As can be seen from testing results in Table 6:
Administration of carbon nanoparticles alone has no effect on growth of cancer cells, and the inhibition rate is 5.35±3.14%.

Administration of carbon nanoparticle-Fe suspension injection alone has a significant inhibition effect on cell growth, and the inhibition rate is 49.87±2.97%.

After combined administration of the carbon nanoparticle-Fe suspension injection and docetaxel at different concentrations, q values are all >1 when the concentration of the docetaxel is ≤1 µg/mL, which shows a synergistic effect on cell growth inhibition (Table 6), indicating that the combined administration of the carbon nanoparticle-Fe suspension injection and the docetaxel can enhance cytotoxicity of the carbon nanoparticles with the ferrous ions adsorbed.

### 7. Combined administration of carbon nanoparticle-Fe suspension injection (CNSI-Fe) and hydroxycamptothecin (HCPT) in vitro:

**Table 7: Inhibition rate (%) and q value of CNSI, CNSI-Fe, HCPT, CNSI-Fe + HCPT against CT26.WT colon cancer in in-vitro cell test**

| Concentration (µg/mL) | Inhibition rate (%) | q value of combined effect | Synergistic effect or not |
|---|---|---|---|
| CNSI 333 | 4.02±1.42 | NA | NA |
| CNSI-Fe 100 | 51.74±1.09 | NA | NA |
| HCPT 4 | 78.14±2.01 | NA | NA |
| HCPT 2 | 72.67±2.78 | NA | NA |
| HCPT 1 | 51.97±2.41 | NA | NA |
| HCPT 0.5 | 34.58±1.95 | NA | NA |
| HCPT 0.25 | 22.79±1.88 | NA | NA |
| HCPT 0.125 | 13.97±3.70 | NA | NA |
| CNSI-Fe 100 + HCPT 4 | 90.14±3.21 | 1.01 | Yes |
| CNSI-Fe 100 + HCPT 2 | 88.12±2.47 | 1.02 | Yes |
| CNSI-Fe 100 + HCPT 1 | 79.64±2.97 | 1.04 | Yes |
| CNSI-Fe 100 + HCPT 0.5 | 74.21±1.42 | 1.08 | Yes |
| CNSI-Fe 100 + HCPT 0.25 | 70.03±1.75 | 1.12 | Yes |
| CNSI-Fe 100 + HCPT 0.125 | 63.48±2.49 | 1.09 | Yes |

As can be seen from testing results in Table 7:
Administration of carbon nanoparticles alone has no effect on growth of cancer cells, and the inhibition rate is 4.02±1.42%.

Administration of carbon nanoparticle-Fe suspension injection alone has a significant inhibition effect on cell growth, and the inhibition rate is 51.74±1.09%.

After combined administration of the carbon nanoparticle-Fe suspension injection and hydroxycamptothecin at different concentrations, q values are all >1 when the concentration of the cisplatin is ≤4 µg/mL, which shows a synergistic effect on cell growth inhibition (Table 7), indicating that the combined administration of the carbon nanoparticle-Fe suspension injection and the hydroxycamptothecin can enhance cytotoxicity of the carbon nanoparticles with the ferrous ions adsorbed.

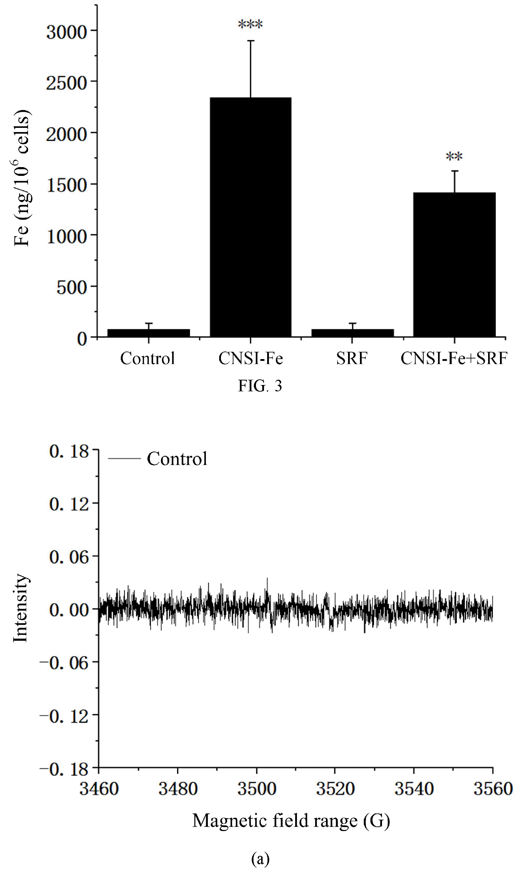

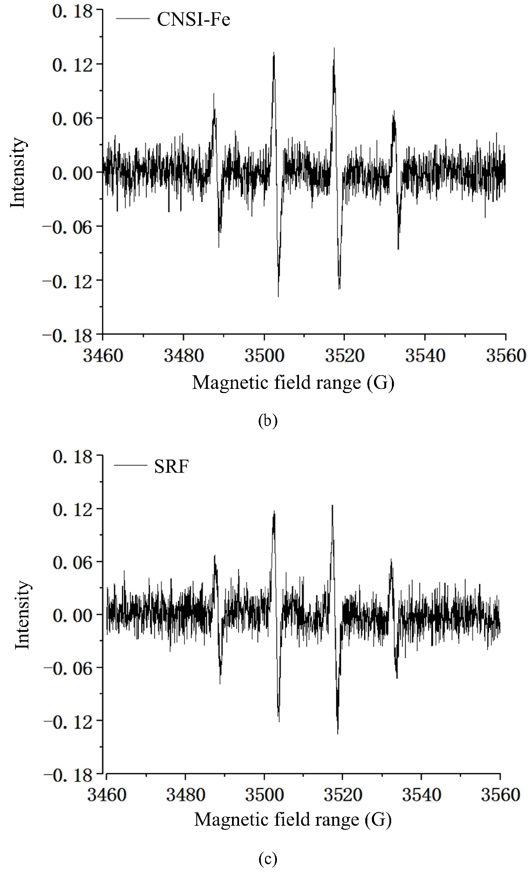

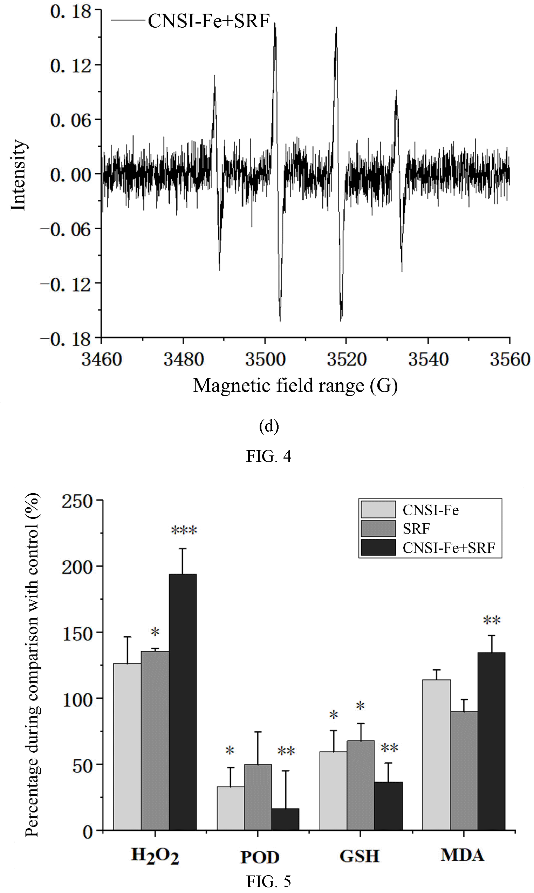

4T1 triple negative breast cancer cells were cultured by the same administration method as in Example 1, and the drugs were grouped into a negative control group, a CNSI-Fe (100 µg/mL) group, an SRF (2 µg/mL) group, and a CNSI-Fe (100µg/mL) + SRF (2µg/mL) group. Cells were collected and the following tests were performed. An antitumor mechanism of CNSI-Fe + SRF on the cells was as follows:
(1) The collected cells were digested by a microwave digestion method, and the iron content of the cells was tested by inductively coupled plasma mass spectrometry (ICP-MS).
   Testing results are shown in FIG. 3, where the intracellular iron content of the CNSI-Fe group and the CNSI-Fe + SRF group is significantly increased, indicating that a large amount of Fe ions enter the cells.
(2) A cell lysis solution was prepared, and was added to each group. After addition of 5,5-dimethyl-1-pyrroline-n-oxide (DMPO), hydroxyl radicals were tested by electron spin resonance spectroscopy (ESR).
   Testing results are shown in FIGS. 4A-4D, characteristic peaks of hydroxyl radicals in the CNSI-Fe group and the SRF group are significantly increased, and a characteristic peak of hydroxyl radicals in the CNSI-Fe + SRF group is higher than that in the CNSI-Fe group and the SRF group.
(3) According to the instructions of hydrogen peroxide (H₂O₂), peroxidase (POD), GSH and malondialdehyde (MDA) assay kits, cell oxidative stress indexes such as H₂O₂, POD, GSH and MDA in the cells were tested.
   FIG. 5 is a graph comparing oxidative stress testing results of the cells in the control group without addition of drugs, the CNSI-Fe administration group, the SRF administration group, and the CNSI-Fe + SRF administration group of the in-vitro cell tests in this example. Testing results are shown in FIG. 5, where POD and GSH are decreased significantly after CNSI-Fe administration; H₂O₂ is increased in a compensatory manner, and GSH is decreased after SRF administration; and H₂O₂ is increased in a compensatory manner, POD and GSH are decreased significantly, and MDA is increased significantly after CNSI-Fe + SRF administration. GSH is a substrate of GPX4, and MDA is a marker product of lipid peroxidation. The results show that the combined administration of CNSI-Fe + SRF further reduces the acting substrate GSH of GPX4, and more MDA is produced.
(4) GPX4 levels were tested by a Western blot method.

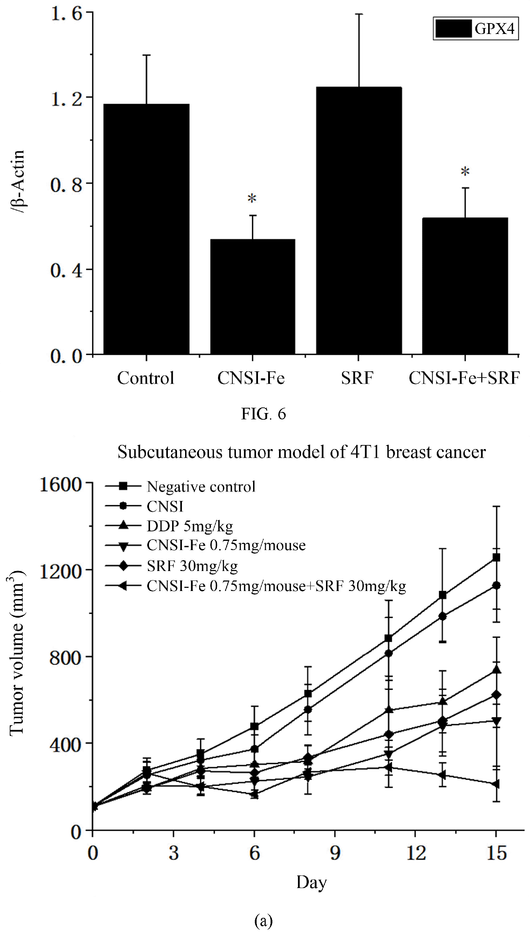

   FIG. 6 is a graph comparing GPX4 levels in the cells of the control group without addition of drugs, the CNSI-Fe administration group, the SRF administration group, and the CNSI-Fe + SRF administration group of the in-vitro cell tests in this example. Testing results are shown in FIG. 6, and GPX4 of the cells is tested by the Western bolt (WB) method. GPX4 has the function of eliminating lipid peroxide, and can be used as one of the indexes to determine ferroptosis of the cells. After administration of CNSI-Fe and CNSI-Fe + SRF, GPX4 is decreased significantly, indicating occurrence of ferroptosis of the cells.

The in-vivo cell test is as follows:
Test materials: murine 4T1 triple negative breast cancer cells, murine CT26.WT colon cancer cells, murine H22 hepatoma cells, human AsPC-1 pancreatic cancer cells, human HT1080 fibrosarcoma cells, RPMI-1640 medium, fetal bovine serum (FBS), cell digestion solution trypsin, penicillin streptomycin mixture, phosphate buffer solution (PBS, pH 7.4), carbon nanoparticle suspension injection, carbon nanoparticle-Fe suspension injection (carbon nanoparticle-ferrous sulfate, CNSI-Fe, concentration being measured in ferrous ions), sorafenib (SRF), doxorubicin (DOX), cisplatin (CDDP), paclitaxel (PTX), gemcitabine (GEM), docetaxel (DTX) and hydroxycamptothecin (HCPT).

Test animals: SPF Balb/c mice, female, 4-6 weeks old, weighing 20±2 g. Balb/c-nu mice, female, 5-7 weeks old, weighing 20±2 g. Free water drinking and eating were carried out during the test. The mice were kept in isolation cages with independent air supply and exposed to light for 12 h a day, and 5 mice were in one cage.

### Test method:

### Example 9

### Combined administration of carbon nanoparticle-Fe suspension injection (CNSI-Fe) and sorafenib (SRF) in vivo:

4T1 cells growing in a logarithmic phase were collected and inoculated into right upper limbs of Balb/c mice with 0.1 mL for each mouse after a concentration of the cell suspension was adjusted to 3×10⁷/mL. After 7 days, the mice with tumor volumes of 100-150 mm³ were grouped into 6 groups with 7 mice in each group.

The 6 groups were a negative control group, a CNSI group, a CDDP 5 mg/kg group, a CNSI-Fe 0.75 mg/mouse group, an SRF 30 mg/kg group, and a CNSI-Fe 0.75 mg/mouse + SRF 30 mg/kg group respectively.
(1) Mice in the negative control group were intratumorally injected with 50 µL of normal saline once a week, twice in total.
(2) Mice in the CNSI group were injected intratumorally with 50 µL of CNSI and 2.5 mg/mouse of carbon nanoparticles once a week, twice in total.
(3) Mice in the CDDP 5 mg/kg group were injected intraperitoneally with CDDP at a dose of 5 mg/kg twice a week, twice in total.
(4) Mice in the CNSI-Fe 0.75 mg/mouse group was injected intratumorally with 50 µL of CNSI-Fe once a week, twice in total, where a concentration of iron ions in CNSI-Fe was 15 mg/mL.
(5) SRF was administered to mice in the SRF 30 mg/kg group by gavage at a dose of 30 mg/kg once daily for 14 days.
(6) Mice in the CNSI-Fe 0.75 mg/mouse + SRF 30 mg/kg group were intratumorally injected with 50 µL of CNSI-Fe (Fe ion concentration of 15 mg/mL) once a week, twice in total, where sorafenib was administered by gavage at a dose of 30 mg/kg one day before CNSI-Fe injection for 14 consecutive days.

An H22 hepatoma model was established by the same method, and administration was performed. The mice were divided into 7 groups with 7 mice in each group, and the groups were a negative control group, a CNSI-Fe 0.375 mg/mouse group, an SRF 15 mg/kg group, an SRF 30 mg/kg group, a CNSI-Fe 0.375 mg/mouse + SRF 15 mg/kg group, a CNSI-Fe 0.375 mg/mouse + SRF 30 mg/kg group, and a CNSI-Fe 0.75 mg/mouse group.

An AsPC-1 pancreatic cancer model was established by the same method, and administration was performed. The mice were divided into 4 groups with 7 mice in each group, and the groups were a negative control group, a CNSI-Fe 0.75 mg/mouse group, an SRF 30mg/kg group, and a CNSI-Fe 0.75 mg/mouse + SRF 30 mg/kg group.

Testing was performed according to groups, and multiple indexes of the mice and tumors were tested:
(1) A tumor volume was measured 3 times a week, where tumor volume = length * width²/2. A tumor growth inhibition rate and a q value were calculated.

In FIG. 7A is a graph comparing tumor volumes of mice in the negative control group, the CNSI control group, the CDDP control group, the CNSI-Fe administration group, the SRF administration group, and the CNSI-Fe + SRF administration group of a 4T1 breast cancer model in the in-vivo cell test in this example. Testing results are shown in FIG. 7A, where the CDDP 5 mg/kg group, the CNSI-Fe 0.75 mg/mouse group, the SRF 30 mg/kg group, and the CNSI-Fe 0.75 mg/mouse + SRF 30 mg/kg group have significant differences compared with the negative control group, which can significantly inhibit the tumor growth.

The CNSI-Fe 0.75 mg/mouse + SRF 30 mg/kg group has a significant difference compared with the CNSI-Fe 0.75 mg/mouse group and the SRF 30 mg/kg group (p<0.01).

The tumor volume inhibition rate on day 15 was calculated. The inhibition rate of the CNSI-Fe 0.75 mg/mouse group is 59.75%, the inhibition rate of the SRF 30 mg/kg group is 50.27%, and the inhibition rate of the CNSI-Fe 0.75 mg/mouse + SRF 30 mg/kg group is the highest, reaching 82.95%, and the q value calculated is 1.04, indicating that a synergistic effect is achieved.

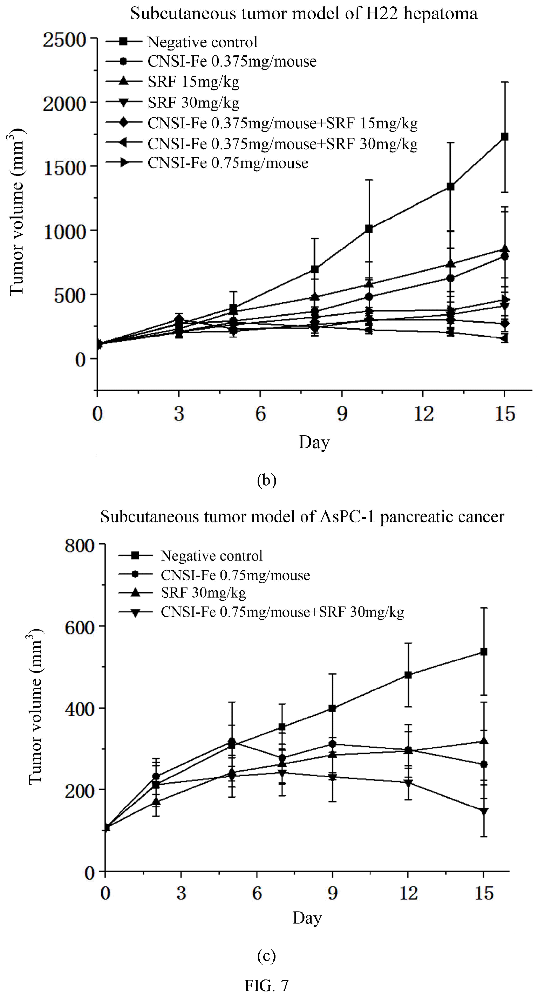

In FIG. 7B is a graph comparing tumor volumes of mice in the negative control group, the CNSI-Fe group, the SRF group, and the CNSI-Fe + SRF group of the H22 hepatoma model in the in-vivo test in this example. Testing results are shown in FIG. 7B, where the tumor volume inhibition rates on day 15 are as follows: the CNSI-Fe 0.375 mg/mouse group is 53.84%, the SRF 15 mg/kg group was 50.59%, the SRF 30 mg/kg group is 76.16%, the CNSI-Fe 0.375 mg/mouse + SRF 15 mg/kg group is 84.25%, the CNSI-Fe 0.375 mg/mouse + SRF 30 mg/kg group is 90.91%, and the CNSI-Fe 0.75 mg/mouse group is 73.40%. Compared with the negative control group, all of these group have significant differences and can significantly inhibit the tumor growth. The CNSI-Fe 0.375 mg/mouse + SRF 15 mg/kg group was significantly different from the CNSI-Fe 0.375 mg/mouse group and the SRF 15 mg/kg group, and the CNSI-Fe 0.375 mg/mouse + SRF 30 mg/kg was significantly different from the CNSI-Fe 0.375 mg/mouse group and the SRF 30 mg/kg group. The q value was calculated. The q values of the CNSI-Fe 0.375 mg/mouse + SRF 15 mg/kg group and the CNSI-Fe 0.375 mg/mouse + SRF 30 mg/kg group are 1.09 and 1.02 respectively, and both of the two groups have a synergistic effect.

In FIG. 7C is a graph comparing tumor volumes of mice in the negative control group, the CNSI-Fe group, the SRF group, and the CNSI-Fe + SRF group of the AsPC-1 pancreatic cancer model in the in-vivo test in this example. Testing results are shown in FIG. 7C, where the tumor volume inhibition rates on day 15 are as follows: 51.21% for the CNSI-Fe 0.75 mg/mouse group, 40.68% for the SRF 30 mg/kg group, and 72.37% for the CNSI-Fe 0.75 mg/mouse + SRF 30 mg/kg group respectively. Compared with the negative control group, these groups have significant differences and can significantly inhibit the tumor growth. The CNSI-Fe 0.75 mg/mouse + SRF 30 mg/kg group is significantly different from the CNSI-Fe 0.75 mg/mouse group and the SRF 30 mg/kg group. A q value was calculated. The q value of the CNSI-Fe 0.75 mg/mouse + SRF 30 mg/kg group is 1.02, indicating that a synergistic effect is achieved.

(2) Hydroxyl radicals in tumor tissues were tested.

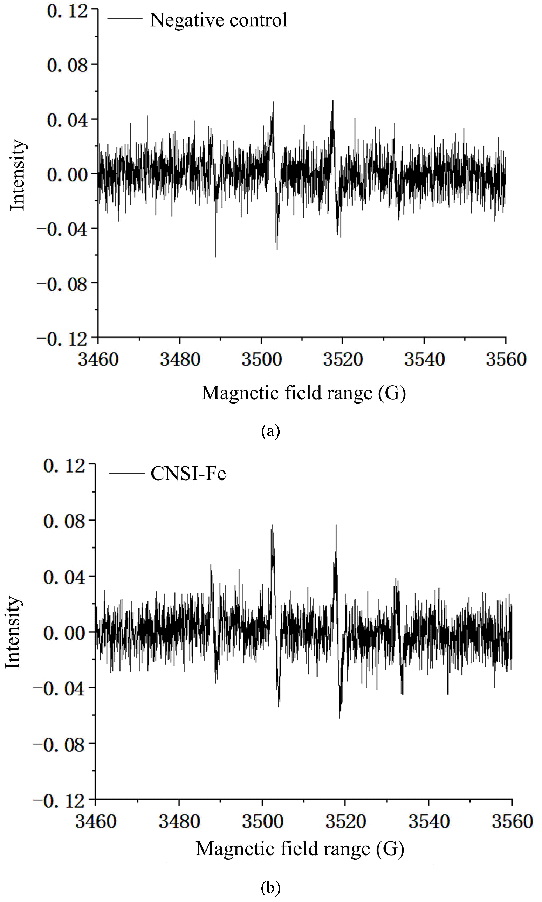

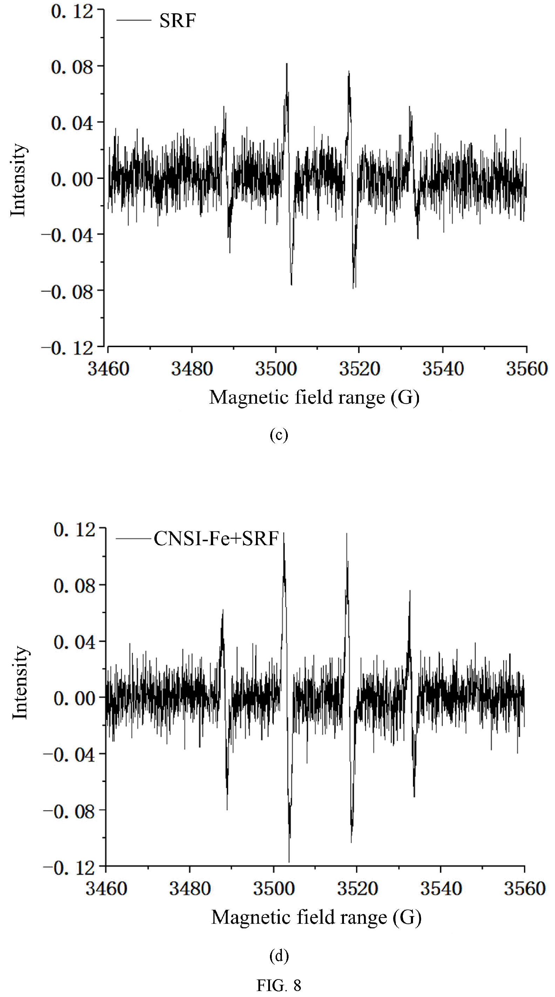

FIGS. 8A-8D are graphs comparing hydroxyl radicals of mouse tumors in the negative control group, the CNSI-Fe administration group, the SRF administration group and the CNSI-Fe + SRF administration group of the 4T1 breast cancer model in the in-vivo test in this example. In FIG. 8A shows hydroxyl radical intensity in the tumor of the negative control group, in FIG. 8B shows hydroxyl radical intensity in the tumor of the CNSI-Fe group, in FIG. 8C shows hydroxyl radical intensity in the tumor of the SRF group, and in FIG. 8D shows hydroxyl radical intensity in the tumor of the CNSI-Fe + SRF group.

Testing results are shown in FIG. 8A, FIG. 8B, FIG. 8C, and FIG. 8D,where characteristic peaks of hydroxyl radicals in the CNSI-Fe group and the SRF group are significantly increased, and a characteristic peak of hydroxyl radicals in the CNSI-Fe + SRF group is higher than that in the CNSI-Fe group and the SRF group.

(3) Concentrations of sorafenib in tumor tissues and tumor cells were tested by HPLC.

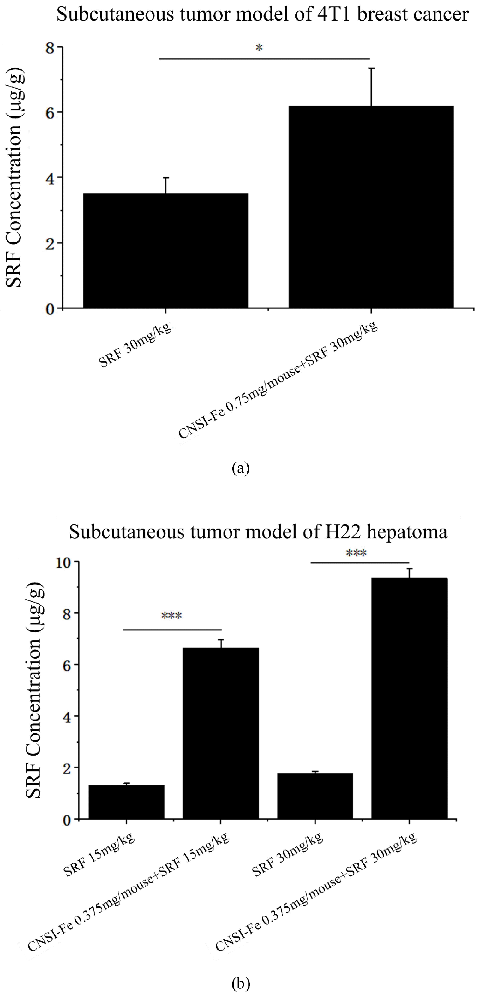

In FIG. 9A is a graph comparing the sorafenib (SRF) content in tumor tissues of the SRF administration group and the CNSI-Fe + SRF administration group of the 4T1 breast cancer model in the in-vivo test in this example. Testing results of the sorafenib concentration in the tumor tissues are shown in FIG. 9A, where the concentration of SRF in the tumor of the SRF 30 mg/kg group is 3.53±0.47 µg/g, and the concentration of SRF in the tumor of the CNSI-Fe 0.75 mg/mouse + SRF 30 mg/kg group is 6.19±1.17 µg/g which is significantly higher than that of SRF administration alone and 1.75 times higher than that of the SRF administration alone, indicating that the carbon nanoparticles with the ferrous ions adsorbed in the tumor can enrich the SRF.

In FIG. 9B is a graph comparing the SRF content in tumor tissues of the SRF administration group and the CNSI-Fe + SRF administration group of the H22 hepatoma model in the in-vivo test in this example. Testing results of the sorafenib concentration in the tumor tissues are shown in FIG. 9B, where the concentration of SRF in the tumor of the SRF 15 mg/kg group is 1.31±0.09 µg/g, and the concentration of SRF in the tumor of the CNSI-Fe 0.375 mg/mouse + SRF 15 mg/kg group is 6.65±0.32 µg/g which is significantly higher than that of SRF administration alone and 5.09 times higher than that of the SRF administration alone. The concentration of SRF in the tumor of the SRF 30 mg/kg group is 1.78±0.08 µg/g, and the concentration of SRF in the tumor of the CNSI-Fe 0.375 mg/mouse + SRF 30 mg/kg group is 9.35±0.37 µg/g which is significantly higher than that of SRF administration alone and 5.26 times higher than that of the SRF administration alone, indicating that the carbon nanoparticles with the ferrous ions adsorbed in the tumor can enrich the SRF.

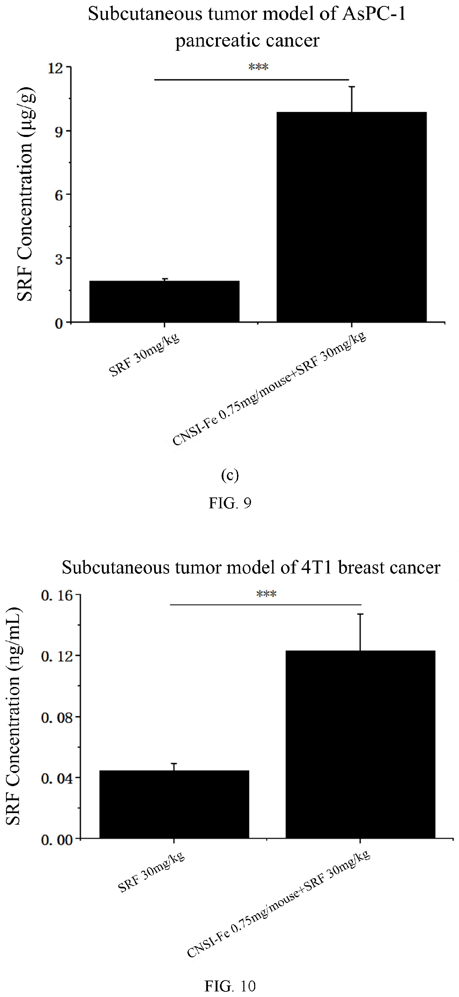

In FIG. 9C is a graph comparing the SRF content in tumor tissues of the SRF administration group and the CNSI-Fe + SRF administration group of the AsPC-1 pancreatic cancer model in the in-vivo test in this example. Testing results of the sorafenib concentration in the tumor tissues are shown in FIG. 9C, where the concentration of SRF in the tumor of the SRF 30 mg/kg group is 1.95±0.10 µg/g, and the concentration of SRF in the tumor of the CNSI-Fe 0.75 mg/mouse + SRF 30 mg/kg group is 9.86±1.21 µg/g which is significantly higher than that of SRF administration alone and 5.06 times higher than that of the SRF administration alone, indicating that the carbon nanoparticles with the ferrous ions adsorbed in the tumor can enrich the SRF.

FIG. 10 is a graph comparing the SRF content in mouse tumor cells of the SRF administration group and the CNSI-Fe + SRF administration group of the 4T1 breast cancer model in the in-vivo test in this example. As shown in FIG. 10, the concentration of SRF in tumor cells (per 10⁴ cells) of the SRF 30 mg/kg group is 0.0447±0.0048 ng/mL, and the concentration of SRF in tumor cells (per 10⁴ cells) of the CNSI-Fe 0.75 mg/mouse + SRF 30 mg/kg group is 0.1233±0.0241 ng/mL which is significantly higher than that of SRF administration alone and is 2.76 times higher than that of the SRF administration alone, indicating that the carbon nanoparticle-Fe suspension injection in the tumor cells can enrich SRF and increase the SRF in the tumor cells.

To sum up, administration of CNSI-Fe and SRF, hydroxyl radicals are produced in the cells, causing ferroptosis in the cells, and significantly inhibiting the tumor growth. The sorafenib is administered before CNSI-Fe, which can produce more hydroxyl radicals, enrich the concentration of the sorafenib in the tumor, enhance an anticancer effect of the carbon nanoparticle-Fe suspension injection and the sorafenib, and have a synergistic effect.

### Example 10

Combined administration of carbon nanoparticle-Fe suspension injection (CNSI-Fe) and doxorubicin (DOX) in vivo:
CT26.WT colon cancer cells growing in a logarithmic phase were collected and inoculated into right upper limbs of Balb/c mice with 0.1 mL for each mouse after a concentration of the cell suspension was adjusted to 3×10⁷/mL. After 7 days, the mice with tumor volumes of 100-150 mm³ were grouped into 7 groups with 7 mice in each group.

The 7 groups were a negative control group, a CNSI-Fe 0.375 mg/mouse group, a DOX 2.5 mg/kg group, a DOX 5 mg/kg group, a CNSI-Fe 0.375 mg/mouse + DOX 2.5 /kg group, a CNSI-Fe 0.375 mg/mouse + DOX 5 mg/kg group, and a CNSI-Fe 0.75 mg/mouse group.
(1) Mice in the negative control group were intratumorally injected with 50 µL of normal saline once a week, twice in total.
(2) Mice in the CNSI-Fe group were injected intratumorally with 50 µL of CNSI-Fe once a week, twice in total, where concentrations of iron ions in CNSI-Fe were 7.5 mg/mL and 15 mg/mL respectively.
(3) DOX was intravenously administered to mice in the DOX groups at doses of 2.5 mg/kg and 5 mg/kg respectively twice weekly, 4 times in total.
(4) Mice in the CNSI-Fe 0.375 mg/mouse + DOX group were intratumorally injected with 50 µL of CNSI-Fe (Fe ion concentration of 7.5 mg/mL) once a week, twice in total, where DOX was administered intravenously at the doses of 2.5 mg/kg and 5 mg/kg day after CNSI-Fe injection twice weekly, 4 times in total.

An HT1080 fibrosarcoma model was established by the same method, and administration was performed. The mice were divided into 4 groups with 7 mice in each group, and the groups were a negative control group, a CNSI-Fe 0.75 mg/mouse group, a DOX 2.5 mg/kg group, and a CNSI-Fe 0.75 mg/mouse + DOX 2.5 mg/kg group.

A tumor volume was measured 2-3 times a week, where tumor volume = length * width ²/2. A tumor growth inhibition rate and a q value were calculated. Tumor tissues were taken, and a concentration of DOX was tested by HPLC.

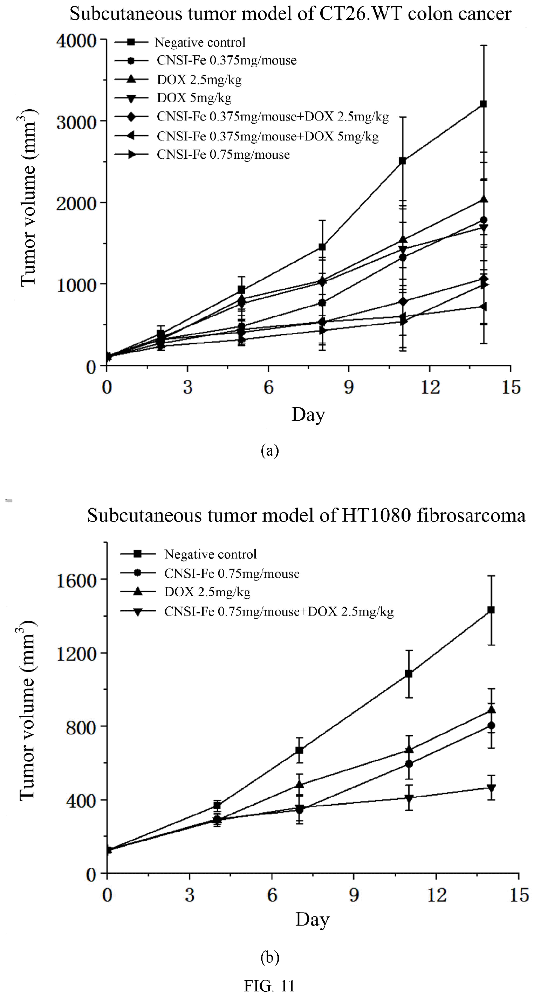

In FIG. 11A is a change graph comparing tumor volumes of subcutaneous tumors of colon cancer after treatment of the negative control group, the CNSI-Fe group, the DOX group, and the CNSI-Fe + DOX group of a CT26.WT colon cancer model in the in-vivo test in this example. As shown in FIG. 11A, the tumor volume inhibition rates on day 14 are calculated, and the inhibition rates of the CNSI-Fe 0.375 mg/mouse group, the DOX 2.5 mg/kg group, the DOX 5 mg/kg group, the CNSI-Fe 0.375 mg/mouse + DOX 2.5 mg/kg, the CNSI-Fe 0.375 mg/mouse + DOX 5 mg/kg group, and the CNSI-Fe 0.75 mg/mouse group are 44.29%, 36.50%, 47.15%, 66.81%, 77.43% and 69.04% respectively. Compared with the negative control group, all of these group have significant differences and can significantly inhibit the tumor growth. The CNSI-Fe 0.375 mg/mouse + DOX 2.5 mg/kg group is significantly different from the CNSI-Fe 0.375 mg/mouse group and the DOX 2.5 mg/kg group, and the CNSI-Fe 0.375 mg/mouse + DOX 5 mg/kg group is significantly different from the CNSI-Fe 0.375 mg/mouse group and the DOX 5 mg/kg group. The q value was calculated. The q values of the CNSI-Fe 0.375 mg/mouse + DOX 2.5 mg/kg group and the CNSI-Fe 0.375 mg/mouse + DOX 5 mg/kg group are 1.03 and 1.10 respectively, and both of the two groups have a synergistic effect.

In FIG. 11B is a change graph comparing tumor volumes of subcutaneous tumors of fibrosarcoma after treatment of the negative control group, the CNSI-Fe group, the DOX group, and the CNSI-Fe + DOX group of the HT1080 fibrosarcoma model in the in-vivo test in this example. As shown in FIG. 11B, the tumor volume inhibition rates on day 14 are calculated as follows: 43.80% for the CNSI-Fe 0.75 mg/mouse group, 38.01% for the DOX 2.5 mg/kg group, and 67.38% for the CNSI-Fe 0.75 mg/mouse + DOX 2.5 mg/kg group respectively. Compared with the negative control group, these groups have significant differences and can significantly inhibit the tumor growth. The CNSI-Fe 0.75 mg/mouse + DOX 2.5 mg/kg group is significantly different from the CNSI-Fe 0.75 mg/mouse group and the DOX 2.5 mg/kg group. A q value was calculated. The q value of the CNSI-Fe 0.75 mg/mouse + DOX 2.5 mg/kg group is 1.03, indicating that a synergistic effect is achieved.

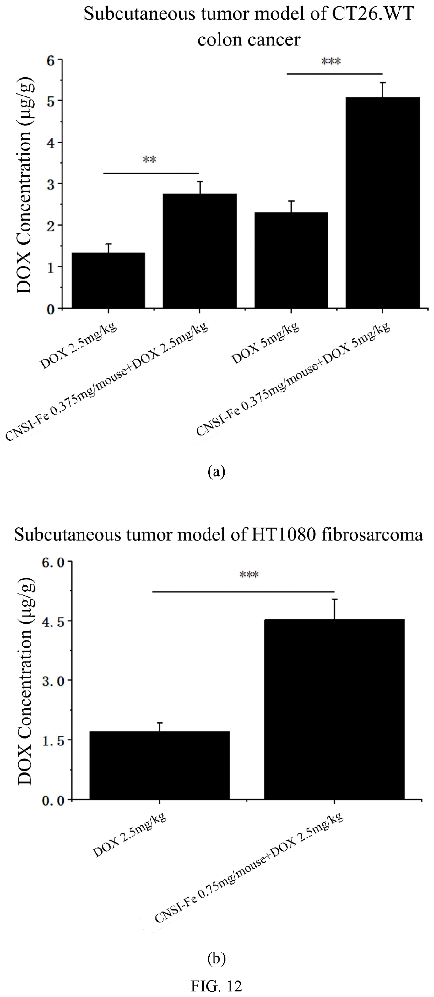

In FIG. 12A shows concentrations of DOX in tumor tissues of subcutaneous tumors of CT26.WT colon cancer of the DOX group and the CNSI-Fe + DOX group in this example. As shown in FIG. 12A, on day 14, the concentration of DOX in the tumor of the DOX 2.5 mg/kg group is 1.34±0.21 µg/g, and the concentration of DOX in the tumor of the CNSI-Fe 0.375 mg/mouse + DOX 2.5 mg/kg group is 2.76±0.30 µg/g which is significantly higher than that of DOX administration alone and 2.06 times higher than that of the DOX administration alone. The concentration of DOX in the tumor of the DOX 5 mg/kg group is 2.31±0.28 µg/g, and the concentration of DOX in the tumor of the CNSI-Fe 0.375 mg/mouse + DOX 5 mg/kg group is 5.09±0.35 µg/g which is significantly higher than that of DOX administration alone and 2.20 times higher than that of the DOX administration alone, indicating that the carbon nanoparticles with the ferrous ions adsorbed in the tumor can enrich the DOX.

In FIG. 12B shows concentrations of DOX in tumor tissues of subcutaneous tumors of HT1080 fibrosarcoma of the DOX group and the CNSI-Fe + DOX group in this example. As shown in FIG. 12B, on day 14, the concentration of DOX in the tumor of the DOX 2.5 mg/kg group is 1.72±0.21 µg/g, and the concentration of DOX in the tumor of the CNSI-Fe 0.75 mg/mouse + DOX 2.5 mg/kg group is 4.53±0.52 µg/g which is significantly higher than that of DOX administration alone and 2.63 times higher than that of the DOX administration alone, indicating that the carbon nanoparticles with the ferrous ions adsorbed in the tumor can enrich the DOX.

### Example 11

### Combined administration of carbon nanoparticle-Fe suspension injection (CNSI-Fe) and cisplatin (CDDP) in vivo:

An H22 hepatoma model was established by the same method as in Example 10, and administration was performed. The mice were divided into 7 groups with 7 mice in each group, and the groups were a negative control group, a CNSI-Fe 0.35 mg/mouse group, a CDDP 2.5 mg/kg group, a CDDP 5 mg/kg group, a CNSI-Fe 0.35 mg/mouse + CDDP 2.5 mg/kg group, a CNSI-Fe 0.35 mg/mouse + CDDP 5 mg/kg group, and a CNSI-Fe 0.75 mg/mouse group. A tumor volume was measured, and an inhibition rate and a q value were calculated. A concentration of CDDP in tumor tissues was tested.

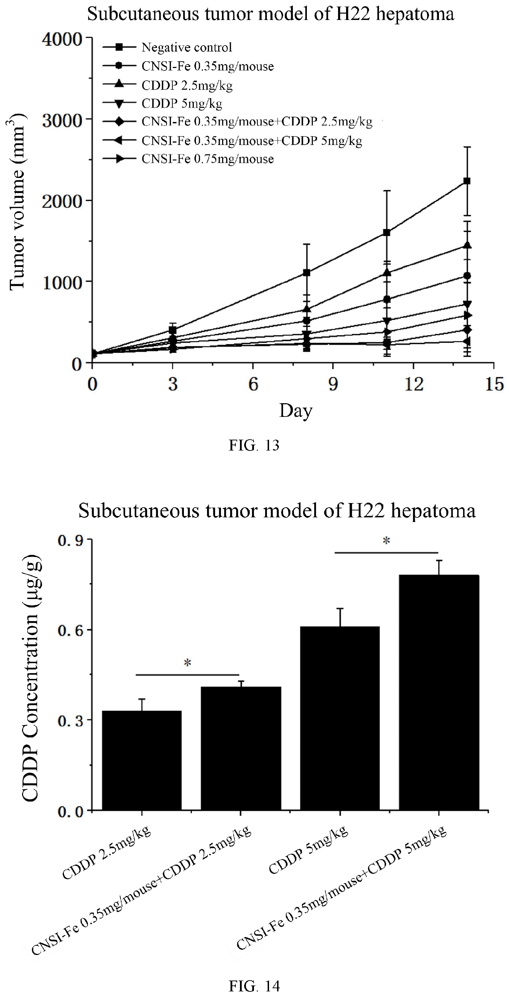

FIG. 13 is a change graph comparing tumor volumes of subcutaneous tumors of hepatoma of the negative control group, the CNSI-Fe group, the CDDP group, and the CNSI-Fe + CDDP group in this example. Testing results are shown in FIG. 13, and the tumor volume inhibition rates on day 14 are calculated follows: 52.14% for the CNSI-Fe 0.35 mg/mouse group, 35.50% for the CDDP 2.5 mg/kg group, 67.62% for the CDDP 5 mg/kg group, 81.97% for the CNSI-Fe 0.35 mg/mouse + CDDP 2.5 mg/kg group, 88.17% for the CNSI-Fe 0.35 mg/mouse + CDDP 5 mg/kg group, and 73.88% for the CNSI-Fe 0.75 mg/mouse group respectively. Compared with the negative control group, all of these group have significant differences and can significantly inhibit the tumor growth. The CNSI-Fe 0.35 mg/mouse + CDDP 2.5 mg/kg group is significantly different from the CNSI-Fe 0.35 mg/mouse group and the CDDP 2.5 mg/kg group, and the CNSI-Fe 0.35 mg/mouse + CDDP 5 mg/kg group is significantly different from the CNSI-Fe 0.35 mg/mouse group and the CDDP 5 mg/kg group. The q value was calculated. The q values of the CNSI-Fe 0.35 mg/mouse + CDDP 2.5 mg/kg group and the CNSI-Fe 0.35 mg/mouse + CDDP 5 mg/kg group are 1.18 and 1.04 respectively, and both of the two groups have a synergistic effect.

FIG. 14 shows concentrations of CDDP in tumor tissues of subcutaneous tumors of hepatoma of the CDDP group and the CNSI-Fe + CDDP group in this example. Testing results are shown in FIG. 14, where the concentration of CDDP in the tumor of the CDDP 2.5 mg/kg group is 0.33±0.04 µg/g, and the concentration of CDDP in the tumor of the CNSI-Fe 0.35 mg/mouse + CDDP 2.5 mg/kg group is 0.41±0.02 µg/g which is significantly higher than that of CDDP administration alone and 1.25 times higher than that of the CDDP administration alone. The concentration of CDDP in the tumor of the CDDP 5 mg/kg group is 0.61±0.06 µg/g, and the concentration of CDDP in the tumor of the CNSI-Fe 0.35 mg/mouse + CDDP 5 mg/kg group is 0.78±0.05 µg/g which is significantly higher than that of CDDP administration alone and 1.28 times higher than that of the CDDP administration alone, indicating that the carbon nanoparticles with the ferrous ions adsorbed in the tumor can enrich the CDDP.

### Example 12

### Combined administration of carbon nanoparticle-Fe suspension injection (CNSI-Fe) and paclitaxel (PTX) in vivo:

A 4T1 breast cancer model was established by the same method as in Example 10, and administration was performed. The mice were divided into 7 groups with 7 mice in each group, and the groups were a negative control group, a CNSI-Fe 0.375 mg/mouse group, a PTX 5 mg/kg group, a PTX 10 mg/kg group, a CNSI-Fe 0.375 mg/mouse + PTX 5mg/kg group, a CNSI-Fe 0.375 mg/mouse + PTX 10mg/kg group, and a CNSI-Fe 0.75 mg/mouse group. A tumor volume was measured, and an inhibition rate and a q value were calculated. A concentration of PTX in tumor tissues was tested.

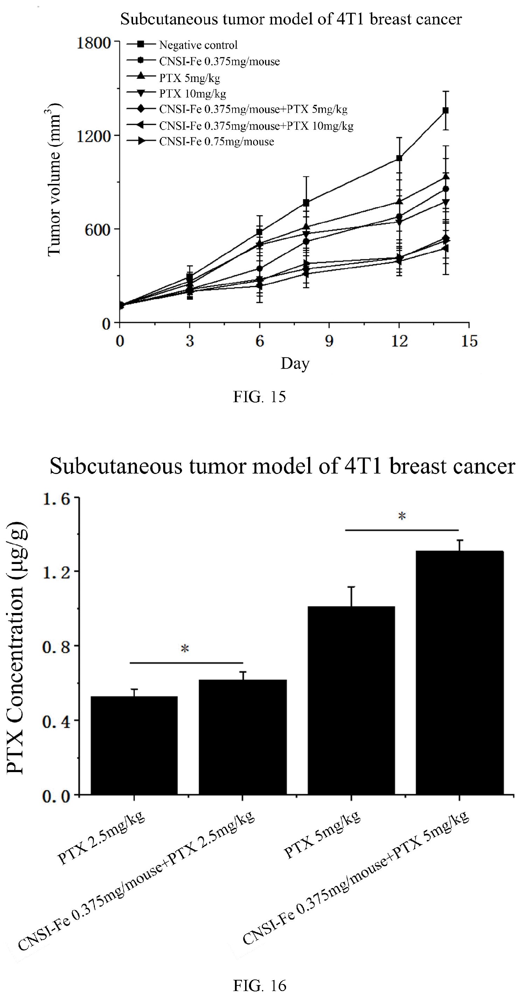

FIG. 15 is a change graph comparing tumor volumes of subcutaneous tumors of breast cancer after treatment of the negative control group, the CNSI-Fe group, the PTX group, and the CNSI-Fe + PTX group in this example. The tumor volume inhibition rates on day 14 are calculated, and the inhibition rates of the CNSI-Fe 0.375 mg/mouse group, the PTX 5 mg/kg group, the PTX 10 mg/kg group, the CNSI-Fe 0.375 mg/mouse + PTX 5 mg/kg, the CNSI-Fe 0.375 mg/mouse + PTX 10 mg/kg group, and the CNSI-Fe 0.75 mg/mouse group are 36.89%, 31.34%, 42.87%, 60.02%, 64.89% and 61.36% respectively. Compared with the negative control group, all of these group have significant differences and can significantly inhibit the tumor growth. The CNSI-Fe 0.375 mg/mouse + PTX 5 mg/kg group is significantly different from the CNSI-Fe 0.375 mg/mouse group and the PTX 5 mg/kg group, and the CNSI-Fe 0.375 mg/mouse + PTX 10 mg/kg group is significantly different from the CNSI-Fe 0.375 mg/mouse group and the PTX 10 mg/kg group. The q value was calculated. The q values of the CNSI-Fe 0.375 mg/mouse + PTX 5 mg/kg group and the CNSI-Fe 0.375 mg/mouse + PTX 10 mg/kg group are 1.06 and 1.01 respectively, and both of the two groups have a synergistic effect.

FIG. 16 shows concentrations of PTX in tumor tissues of subcutaneous tumors of breast cancer of the PTX group and the CNSI-Fe + PTX group in this example. Testing results are shown in FIG. 16, where the concentration of PTX in the tumor of the PTX 5 mg/kg group is 0.53±0.04 µg/g, and the concentration of PTX in the tumor of the CNSI-Fe 0.375 mg/mouse + PTX 5 mg/kg group is 0.62±0.04 µg/g which is significantly higher than that of PTX administration alone and 1.17 times higher than that of the PTX administration alone. The concentration of PTX in the tumor of the PTX 10 mg/kg group is 1.01±0.11 µg/g, and the concentration of PTX in the tumor of the CNSI-Fe 0.375 mg/mouse + PTX 10 mg/kg group is 1.31±0.06 µg/g which is significantly higher than that of PTX administration alone and 1.30 times higher than that of the PTX administration alone, indicating that the carbon nanoparticles with the ferrous ions adsorbed in the tumor can enrich the PTX.

### Example 13

### Combined administration of carbon nanoparticle-Fe suspension injection (CNSI-Fe) and gemcitabine (GEM) in vivo:

An AsPC-1 pancreatic cancer model was established by the same method as in Example 10, and administration was performed. The mice were divided into 5 groups with 7 mice in each group, and the groups were a negative control group, a CNSI-Fe 0.375 mg/mouse group, a GEM 120 mg/kg group, a CNSI-Fe 0.375 mg/mouse + GEM 120 mg/kg group, and a CNSI-Fe 0.75 mg/mouse group. A tumor volume was measured, and an inhibition rate and a q value were calculated. A concentration of GEM in tumor tissues was tested.

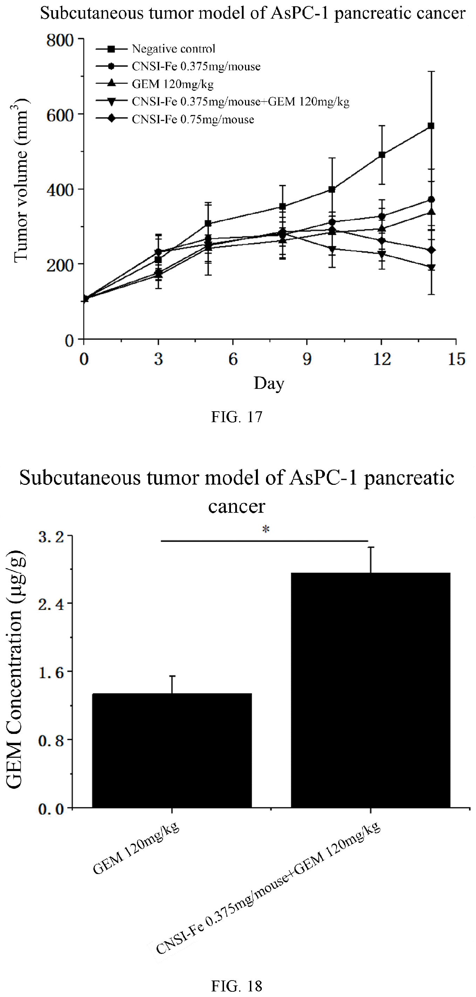

FIG. 17 is a change graph comparing tumor volumes of subcutaneous tumors of pancreatic cancer of the negative control group, the CNSI-Fe group, the GEM group, and the CNSI-Fe + GEM group in this example. Testing results are shown in FIG. 17, and the tumor volume inhibition rates on day 14 are calculated as follows: 34.42% for the CNSI-Fe 0.375 mg/mouse group, 40.29% for the GEM 120 mg/kg group, 66.08% for the CNSI-Fe 0.375 mg/mouse + GEM 120 mg/kg group, and 58.06% for the CNSI-Fe 0.75 mg/mouse group respectively. Compared with the negative control group, these groups have significant differences and can significantly inhibit the tumor growth. The CNSI-Fe 0.375 mg/mouse + GEM 120 mg/kg group is significantly different from the CNSI-Fe 0.375 mg/mouse group and the GEM 120 mg/kg group. A q value was calculated. The q value of the CNSI-Fe 0.375 mg/mouse + GEM 120 mg/kg group is 1.09, indicating that a synergistic effect is achieved.

FIG. 18 shows concentrations of GEM in tumor tissues of subcutaneous tumors of pancreatic cancer of the GEM group and the CNSI-Fe + GEM group in this example. Testing results of the concentration of GEM in the tumor tissues are shown in FIG. 18, where the concentration of GEM in the tumor of the GEM 120 mg/kg group is 5.62±0.26 µg/g, and the concentration of GEM in the tumor of the CNSI-Fe 0.375 mg/mouse + GEM 120 mg/kg group is 6.19±0.16 µg/g which is significantly higher than that of GEM administration alone and 1.10 times higher than that of the GEM administration alone, indicating that the carbon nanoparticles with the ferrous ions adsorbed in the tumor can enrich the GEM.

### Example 14

### Combined administration of carbon nanoparticle-Fe suspension injection (CNSI-Fe) and docetaxel (DTX) in vivo:

A 4T1 breast cancer model was established by the same method as in Example 10, and administration was performed. The mice were divided into 7 groups with 7 mice in each group, and the groups were a negative control group, a CNSI-Fe 0.375 mg/mouse group, a DTX 5 mg/kg group, a DTX 10 mg/kg group, a CNSI-Fe 0.375 mg/mouse + DTX 5 mg/kg group, a CNSI-Fe 0.375 mg/mouse + DTX 10 mg/kg group, and a CNSI-Fe 0.75 mg/mouse group. A tumor volume was measured, and an inhibition rate and a q value were calculated. A concentration of DTX in tumor tissues was tested.

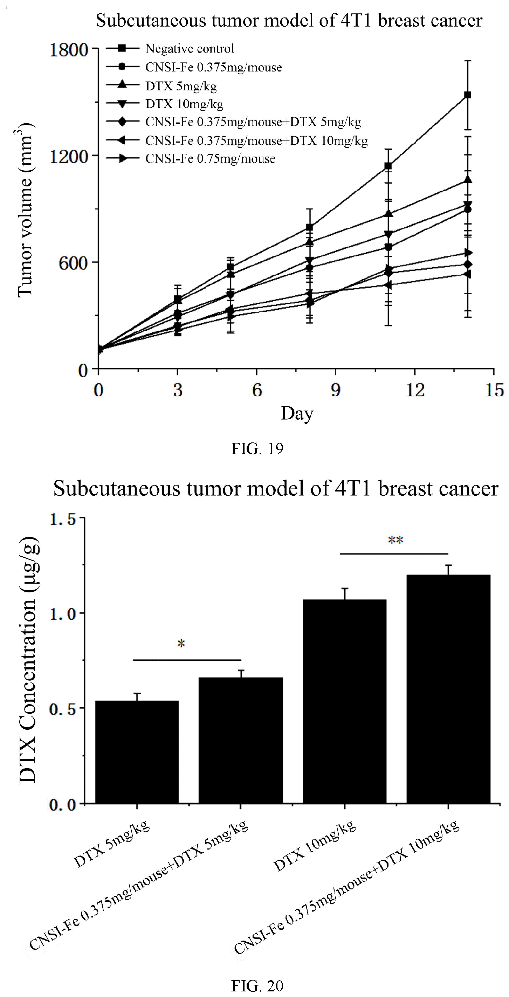

FIG. 19 is a change graph comparing tumor volumes of subcutaneous tumors of breast cancer after treatment of the negative control group, the CNSI-Fe group, the DTX group, and the CNSI-Fe + DTX group in this example. Testing results are shown in FIG. 19, and the tumor volume inhibition rates on day 14 are calculated follows: 41.74% for the CNSI-Fe 0.375 mg/mouse group, 31.07% for the DTX 5 mg/kg group, 39.75% for the DTX 10 mg/kg group, 61.75% for the CNSI-Fe 0.375 mg/mouse + DTX 5 mg/kg group, 65.32% for the CNSI-Fe 0.375 mg/mouse + DTX 10 mg/kg group, and 57.51% for the CNSI-Fe 0.75 mg/mouse group respectively. Compared with the negative control group, all of these group have significant differences and can significantly inhibit the tumor growth. The CNSI-Fe 0.375 mg/mouse + DTX 5 mg/kg group is significantly different from the CNSI-Fe 0.375 mg/mouse group and the DTX 5 mg/kg group, and the CNSI-Fe 0.375 mg/mouse + DTX 10 mg/kg group is significantly different from the CNSI-Fe 0.375 mg/mouse group and the DTX 10 mg/kg group. The q value was calculated. The q values of the CNSI-Fe 0.375 mg/mouse + DTX 5 mg/kg group and the CNSI-Fe 0.375 mg/mouse + DTX 10 mg/kg group are 1.03 and 1.01 respectively, and both of the two groups have a synergistic effect.

FIG. 20 shows concentrations of DTX in tumor tissues of subcutaneous tumors of breast cancer in the DTX group and the CNSI-Fe + DTX group. Testing results are shown in FIG. 20, where the concentration of DTX in the tumor of the DTX 5 mg/kg group is 0.54±0.04 µg/g, and the concentration of DTX in the tumor of the CNSI-Fe 0.375 mg/mouse + DTX 5 mg/kg group is 0.66±0.04 µg/g which is significantly higher than that of DTX administration alone and 1.22 times higher than that of the DTX administration alone. The concentration of DTX in the tumor of the DTX 10 mg/kg group is 1.07±0.06 µg/g, and the concentration of DTX in the tumor of the CNSI-Fe 0.375 mg/mouse + DTX 10 mg/kg group is 1.20±0.05 µg/g which is significantly higher than that of DTX administration alone and 1.12 times higher than that of the DTX administration alone, indicating that the carbon nanoparticles with the ferrous ions adsorbed in the tumor can enrich the DTX.

### Example 15

### Combined administration of carbon nanoparticle-Fe suspension injection (CNSI-Fe) and hydroxycamptothecin (HCPT) in vivo:

A CT26.WT colon cancer model was established by the same method as in Example 10, and administration was performed. The mice were divided into 7 groups with 7 mice in each group, and the groups were a negative control group, a CNSI-Fe 0.375 mg/mouse group, a HCPT 5 mg/kg group, a HCPT 10 mg/kg group, a CNSI-Fe 0.375 mg/mouse + HCPT 5 mg/kg group, a CNSI-Fe 0.375 mg/mouse + HCPT 10 mg/kg group, and a CNSI-Fe 0.75 mg/mouse group. A tumor volume was measured, and an inhibition rate and a q value were calculated. A concentration of HCPT in tumor tissues was tested.

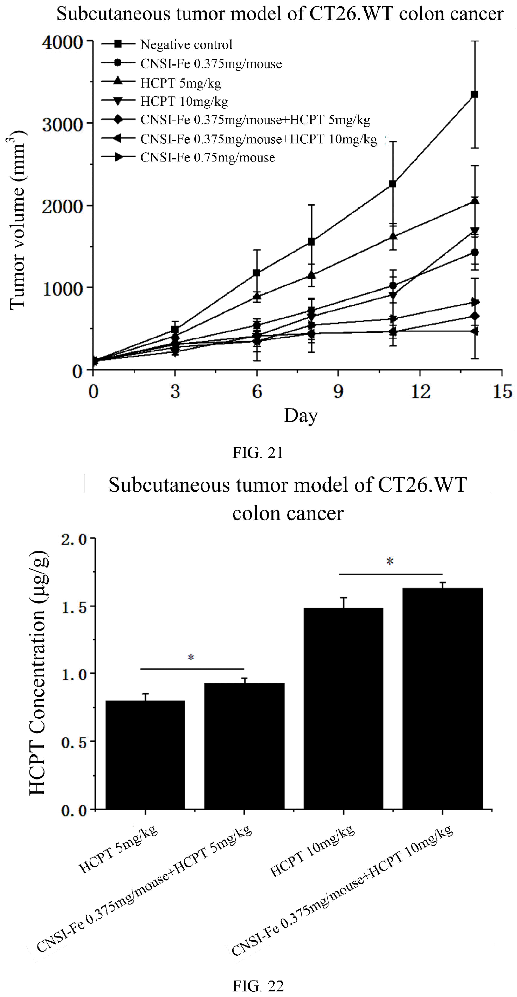

FIG. 21 is a change graph comparing tumor volumes of subcutaneous tumors of colon cancer of the negative control group, the CNSI-Fe group, the HCPT group, and the CNSI-Fe + HCPT group in this example. Testing results are shown in FIG. 21, and the tumor volume inhibition rates on day 14 are calculated follows: 57.28% for the CNSI-Fe 0.375 mg/mouse group, 38.72% for the HCPT 5 mg/kg group, 49.33% for the HCPT 10 mg/kg group, 80.44% for the CNSI-Fe 0.375 mg/mouse + HCPT 5 mg/kg group, 85.92% for the CNSI-Fe 0.375 mg/mouse + HCPT 10 mg/kg group, and 75.31% for the CNSI-Fe 0.75 mg/mouse group respectively. Compared with the negative control group, all of these group have significant differences and can significantly inhibit the tumor growth. The CNSI-Fe 0.375 mg/mouse + HCPT 5 mg/kg group is significantly different from the CNSI-Fe 0.375 mg/mouse group and the HCPT 5 mg/kg group, and the CNSI-Fe 0.375 mg/mouse + HCPT 10 mg/kg group is significantly different from the CNSI-Fe 0.375 mg/mouse group and the HCPT 10 mg/kg group. The q value was calculated. The q values of the CNSI-Fe 0.375 mg/mouse + HCPT 5 mg/kg group and the CNSI-Fe 0.375 mg/mouse + HCPT 10 mg/kg group are 1.09 and 1.10 respectively, and both of the two groups have a synergistic effect.

FIG. 22 shows concentrations of HCPT in tumor tissues of subcutaneous tumors of colon cancer in the HCPT group and the CNSI-Fe + HCPT group. Testing results are shown in FIG. 22, where the concentration of HCPT in the tumor of the HCPT 5 mg/kg group is 0.80±0.05 µg/g, and the concentration of HCPT in the tumor of the CNSI-Fe 0.375 mg/mouse + HCPT 5 mg/kg group is 0.93±0.04 µg/g which is significantly higher than that of HCPT administration alone and 1.22 times higher than that of the HCPT administration alone. The concentration of HCPT in the tumor of the HCPT 10 mg/kg group is 1.48±0.08 µg/g, and the concentration of HCPT in the tumor of the CNSI-Fe 0.375 mg/mouse + HCPT 10 mg/kg group is 1.63±0.04 µg/g which is significantly higher than that of HCPT administration alone and 1.12 times higher than that of the HCPT administration alone, indicating that the carbon nanoparticles with the ferrous ions adsorbed in the tumor can enrich the HCPT.

### Example 16

### Combined administration of carbon nanoparticle-Fe suspension injection (CNSI-Fe), paclitaxel (PTX), and gemcitabine (GEM) in vivo:

An AsPC-1 pancreatic cancer model was established by the same method as in Example 10, and administration was performed. The mice were divided into 5 groups with 7 mice in each group, and the groups were a negative control group, a CNSI-Fe 0.75 mg/mouse group, a PTX 20 mg/kg + GEM 120 mg/kg group, and a CNSI-Fe 0.75 mg/mouse + PTX 20 mg/kg + GEM 120 mg/kg group. A tumor volume was measured, and an inhibition rate and a q value were calculated.

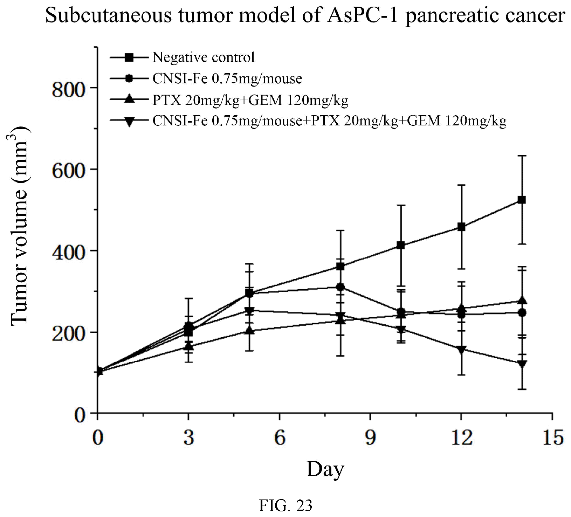

FIG. 23 is a change graph comparing tumor volumes of pancreatic cancer subcutaneous tumors of the negative control group, the CNSI-Fe group, the PTX + GEM group, and the CNSI-Fe + PTX + GEM group in this example. Testing results are shown in FIG. 23, and the tumor volume inhibition rates on day 14 are calculated as follows: 52.71% for the CNSI-Fe 0.75 mg/mouse group, 47.30% for the PTX 20 mg/kg + GEM 120 mg/kg group, and 76.63% for the CNSI-Fe 0.75 mg/mouse + PTX 20 mg/kg + GEM 120 mg/kg group respectively. Compared with the negative control group, these groups have significant differences and can significantly inhibit the tumor growth. The CNSI-Fe 0.75 mg/mouse + PTX 20 mg/kg + GEM 120 mg/kg group is significantly different from the CNSI-Fe 0.75 mg/mouse group and the PTX 20 mg/kg + GEM 120 mg/kg group. A q value was calculated. The q value of the CNSI-Fe 0.75 mg/mouse + PTX 20 mg/kg + GEM 120 mg/kg group is 1.02, indicating that a synergistic effect is achieved.

It should be understood that particular embodiments described above are merely used for illustratively describing or explaining the principle of the present application, rather than constituting limitings to the present application. Therefore, any modification, equivalent substitution, improvement, etc. made without departing from the spirit and the scope of the present application should fall within the protection scope of the present application. In addition, the claims appended of the present application are intended to cover all changes and modifications that fall within the scope and boundaries of the appended claims, or equivalents of such ranges and boundaries.

## Claims

1. Carbon nanoparticle-Fe suspension injection for use in inhibiting tumor growth, wherein the carbon nanoparticle-Fe suspension injection is administered in combination with an anticancer drug, the anticancer drug is administered orally or intravenously, and the carbon nanoparticle-Fe suspension injection is administered through intratumoral injection.

2. The carbon nanoparticle-Fe suspension injection for use according to claim 1, wherein the anticancer drug is one or more selected from sorafenib, doxorubicin, cisplatin, paclitaxel, gemcitabine, docetaxel and hydroxycamptothecin.

3. The carbon nanoparticle-Fe suspension injection for use according to claim 2, wherein the combined administration of the carbon nanoparticle-Fe suspension injection and the anticancer drug is for use in treating pancreatic cancer, lung cancer, gastric cancer, colorectal cancer, breast cancer, cervical cancer, hepatoma, thyroid cancer, ovarian cancer or sarcoma.

4. The carbon nanoparticle-Fe suspension injection for use according to any one of claims 1-3, wherein the carbon nanoparticle-Fe suspension injection is prepared by mixing carbon nanoparticle suspension injection with ferrous sulfate for injection, a concentration of carbon nanoparticles in the carbon nanoparticle suspension injection is 20-100 mg/mL, and a concentration of ferrous ions in the carbon nanoparticle-Fe suspension injection is 0.1-60 mg/mL.

5. The carbon nanoparticle-Fe suspension injection for use according to claim 4, wherein a concentration of carbon nanoparticles in the carbon nanoparticle suspension injection is 50 mg/mL, and the concentration of the ferrous ions in the carbon nanoparticle-Fe suspension injection is 7.0-60 mg/mL.

6. The carbon nanoparticle-Fe suspension injection for use according to claim 4, wherein the concentration of the ferrous ions in the carbon nanoparticle-Fe suspension injection is 30 mg/mL or 60 mg/mL.

7. The carbon nanoparticle-Fe suspension injection for use according to claim 5 or 6, wherein the carbon nanoparticles loaded with ferrous ions have a particle size of 90-250 nm, and the carbon nanoparticle-Fe suspension injection has a pH value of 2.0-6.0.

8. The carbon nanoparticle-Fe suspension injection for use according to any one of claims 1-7, wherein the combined administration of the carbon nanoparticle-Fe suspension injection and the anticancer drug is performed for 1 to 6 cycles, and proportions within a combined administration cycle are as follows:
the carbon nanoparticle-Fe suspension injection is administered once every 14 days, with a ferrous ion dose of 30 mg to 150 mg per injection;
proportions of the anticancer drug comprise:
the sorafenib is administered twice daily with 0.2 g-0.4 g each time; or
the doxorubicin is administered once every three weeks with 1.2-2.4 mg/kg or 30-75 mg/m² each time; or
the cisplatin is administered once every four weeks with 50-120 mg/m² each time, or once daily with 15-20 mg/m² each time for five consecutive days; or
the paclitaxel is administered once weekly with 80 mg/m² each time, or once every three to four weeks with 135-175 mg/m² each time; or
the gemcitabine is administered once weekly with 1000-1250 mg/m² each time; or
the docetaxel is administered once every three weeks with 75 mg/m² each time; or
the hydroxycamptothecin is administered once daily with 4-6 mg each time.
